(19) Europäisches Patentamt
European Patent Office
Office européen des brevets

(11) EP 2 845 851 A1

(12) **EUROPEAN PATENT APPLICATION**
published in accordance with Art. 153(4) EPC

(43) Date of publication:
**11.03.2015 Bulletin 2015/11**

(21) Application number: **13784541.8**

(22) Date of filing: **12.04.2013**

(51) Int Cl.:
***C07D 211/26*** *(2006.01)* ***A01N 43/40*** *(2006.01)*
***A01P 7/02*** *(2006.01)* ***A01P 7/04*** *(2006.01)*
***C07D 401/04*** *(2006.01)* ***C07D 401/12*** *(2006.01)*
***C07D 401/14*** *(2006.01)*

(86) International application number:
**PCT/JP2013/061559**

(87) International publication number:
**WO 2013/164954 (07.11.2013 Gazette 2013/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.05.2012 JP 2012104686**

(71) Applicant: **Sumitomo Chemical Company Limited**
**Tokyo 104-8260 (JP)**

(72) Inventor: **ARIMORI, Sadayuki**
**Takarazuka-shi**
**Hyogo 665-8555 (JP)**

(74) Representative: **Vossius & Partner**
**Siebertstrasse 3**
**81675 München (DE)**

(54) **PIPERIDINE COMPOUND AND PEST-CONTROL USE THEREFORE**

(57) A piperidine compound represented by the following Formula (1):

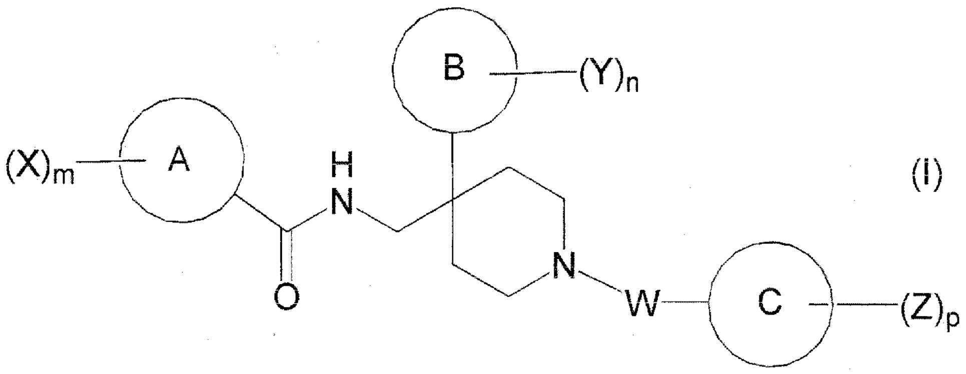

(I)

wherein ring A, ring B, and ring C are the same or differen t and each represent a benzene ring or a nitrogen-containing heteroaromatic ring; W represents a C1-C4 alkylene or alkenylene group; X, Y, and Z are the same o r different and each represent a halogen atom, a C1-C6 alkyl group, a halo C1-C6 alkyl group, a C1-C6 alkoxy group, or a halo C1-C6 alkoxy group; m, n, and p are the same or different and each represent an integer of 0 to 5; when m is greater than or equal two 2, the Xs are t he same or different; when n is greater than or equal to 2, the Ys are the same or different; and when p is greater th an or equal to 2, the Zs are the same or different has an excellent controlling efficacy against pests.

EP 2 845 851 A1

## Description

### Technical Field

The present invention relates to a piperidine compound and its use in pest control.

### Background Art

Heretofore, various pest control agents for controlling pests have been developed and have been practically used.

A compound having various phenylalkylpiperazine skeletons has been reported in Arch. Pharm. (Weinheim) 319, 505-515 (1986), but there is no disclosure relating to the application thereof for controlling pests.

### Summary of Invention

An object of the present invention is to provide a novel compound which has excellent controlling efficacy against pests.

The present invention provides a piperidine compound (hereinafter, also referred to as a compound of the present invention) represented by the following Formula (I), and its use in pest cotrol.

[Item 1]

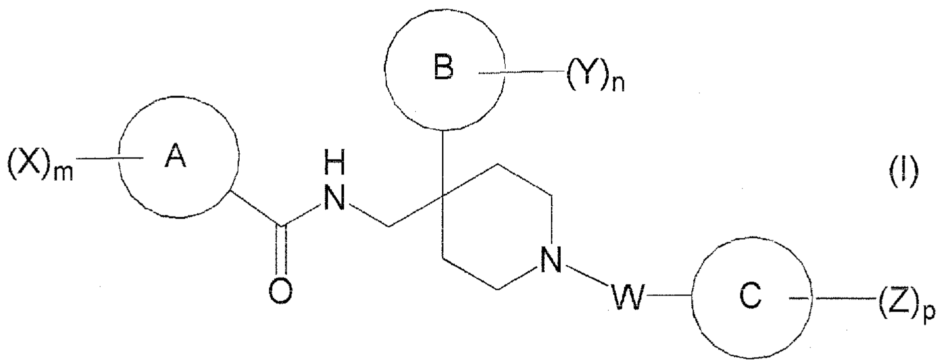

(I)

A piperidine compound represented by Formula (I) wherein, ring A, ring B, and ring C are the same or differe nt and each represent a benzene ring or a nitrogen-containing heteroaromatic ring; W represents a C1-C4 alkylene or alkenylene group; X, Y, and Z are the same or d ifferent and each represent a halogen atom, a C1-C6 alkyl group, a halo C1-C6 alkyl group, a C1-C6 alkoxy group, or a halo C1-C6 alkoxy group; m, n, and p are the same or differe nt and each represent an integer of 0 to 5; when m is greater than or equal to 2, the Xs are the same or different; when n is greater than or equal to 2, the Ys are the same or different; and when p is greater than or equal to 2, the Zs are the same or differen.

[Item 2] A pest control agent comprising the piperidine compound according to [Item 1] and an inert carrier.
[Item 3] A method for controlling pests comprising applying an effective amount of the piperidine compound according to [Item 1] to pests or a habitat of the pests.

### Description of Embodiments

The compound of the present invention is a piperidine compound represented by Formula (I). In some cases, an isomer derived from an asymmetric carbon atom exists in the compound of the present invention. However, in the present invention, the isomer having pest control activity and a mixture of isomers in an arbitrary proportion are included.

Hereinafter, examples thereof will be described using terms used in the present specification.

In the present invention, examples of the "nitrogen-containing heteroaromatic ring" include a 5- or 6-membered monocyclic heteroaromatic ring containing 1 to 3 nitrogen atoms, and specific examples thereof include a pyrrole ring, an imidazole ring, a pyrazole ring, a pyridine ring, a pyrazine ring, a pyrimidine ring, and a pyridazine ring.

In the present invention, examples of the "C1-C4 alkylene or alkenylene group" include a methylene group, an ethylene group, a trimethylene group (propane-1,3-diyl group), a propenylene group (propene-1,3-diyl group), and a

tetramethylene group (butane-1,4-diyl group).

In the present invention, examples of the "halogen atom" include a fluorine atom, a chlorine atom, and a bromine atom.

In the present invention, examples of the "C1-C6 alkyl group" include a methyl group, an ethyl group, a propyl group, an isopropyl group, a butyl group, a sec-butyl group, an isobutyl group, a tert-butyl group, a pentyl group, and a hexyl group. Preferred examples thereof include C1-C3 alkyl groups, such as the methyl group, the ethyl group, the propyl group, and the isopropyl group.

In the present invention, examples of the "halo C1-C6 alkyl group" include groups in which one or more hydrogen atoms of the above-described "C1-C6 alkyl group" have been substituted with halogen atoms (for example, a fluorine atom, a chlorine atom, and a bromine atom), for example, a fluoromethyl group, a difluoromethyl group, a trifluoromethyl group, a chloromethyl group, a dichloromethyl group, a trichloromethyl group, a chlorodifluoromethyl group, a 2,2,2-trifluoroethyl group, a 1,1,2,2-tetrafluoroethyl group, and a pentafluoroethyl group.

In the present invention, examples of the "C1-C6 alkoxy group" include a methoxy group, an ethoxy group, a propoxy group, an isopropoxy group, a butoxy group, a sec-butoxy group, an isobutoxy group, a tert-butoxy group, a pentyloxy group, and a hexyloxy group.

In the present invention, examples of the "halo C1-C6 alkoxy group" include groups in which one or more hydrogen atoms of the above-described "C1-C6 alkoxy group" have been substituted with halogen atoms (for example, a fluorine atom, a chlorine atom, and a bromine atom), for example, a fluoromethoxy group, a difluoromethoxy group, a trifluoromethoxy group, a chloromethoxy group, a dichloromethoxy group, a trichloromethoxy group, a 2,2,2-trifluoroethoxy group, a 1,1,2,2-tetrafluoroethoxy group, and a pentafluoroethoxy group.

Preferred examples of X include halogen atoms (for example, a fluorine atom and a chlorine atom), C1-C3 alkyl groups (for example, a methyl group and an ethyl group), halo C1-C3 alkyl groups (for example, a trifluoromethyl group and a pentafluoroethyl group), and C1-C3 alkoxy groups (for example, a methoxy group and an ethoxy group).

Preferred examples of Y include halogen atoms (for example, a fluorine atom and a chlorine atom), C1-C3 alkyl groups (for example, a methyl group and an ethyl group), halo(C1-C3)alkyl groups (for example, a trifluoromethyl group and a pentafluoroethyl group), and C1-C3 alkoxy groups (for example, a methoxy group and an ethoxy group).

Preferred examples of Z include halogen atoms (for example, a fluorine atom and a chlorine atom), C1-C3 alkyl groups (for example, a methyl group and an ethyl group), halo C1-C3 alkyl groups (for example, a trifluoromethyl group and a pentafluoroethyl group), and C1-C3 alkoxy groups (for example, a methoxy group and an ethoxy group).

m is an integer of 0 to 5 and is preferably 0, 1, 2, 3, or 4. When m is greater than or equal to 2, Xs may be the same or different.

n is an integer of 0 to 5 and is preferably 0, 1, 2, or 3, and more preferably 0, 1, or 2. When n is greater than or equal to 2, Ys may be the same or different.

p is an integer of 0 to 5 and is preferably 0, 1, 2, or 3, and more preferably 0, 1, or 2. When p is greater than or equal to 2, Zs may be the same or different.

In the piperidine compound represented by Formula (I), as specific examples of a group represented by the following Formula:

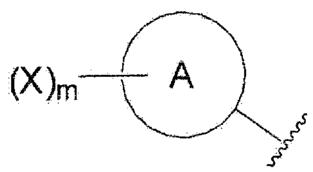

wherein, ring A, X, and m are as defined above, there are groups represented by the following Formulas:

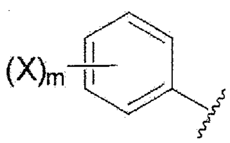
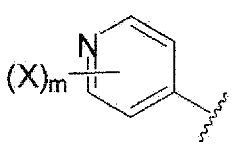
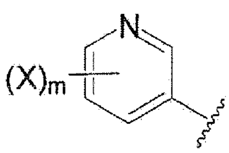
or
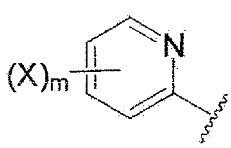

wherein, X and m are as defined above.

More specific examples thereof include a phenyl group, a 2-halo(for example, 2-F or 2-Cl)phenyl group, a 3-halo(for example, 3-F or 3-Cl)phenyl group, a 4-halo(for example, 4-F or 4-Cl)phenyl group, a 3,5-dihalo(for example, 3,5-di-F or 3,5,di-Cl)phenyl group, a 2,4,6-trihalo(for example, 2,4,6-tri-F or 2,4,6-tri-Cl)phenyl group, a 2,3,5,6-tetrahalo(for example, 2,3,5,6-tetra-F or 2,3,5,6-tetra-Cl)phenyl group, a 1-methoxyphenyl group, a 2-methoxyphenyl group, a 3-methoxyphenyl group, a 2,6-dimethoxyphenyl group, a 1-trifluoromethoxyphenyl group, a 2-trifluoromethoxyphenyl

group, and a 3-trifluoromethoxyphenyl group; and a 2-pyridyl group, a 3-pyridyl group, a 4-pyridyl group, a 2-halo(for example, 2-F or 2-Cl)pyridin-4-yl group, a 2,6-dihalo(for example, 2,6-di-F or 2,6-di-Cl)pyridin-4-yl group, a 3-halo(for example, 3-F or 3-Cl)pyridin-2-yl group, and a 2-halo(for example, 2-F or 2-Cl)pyridin-3-yl group.

In the piperidine compound represented by Formula (I), as specific examples of a group represented by the following Formula:

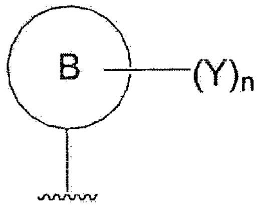

wherein, ring B, Y, and n are as defined above, there are groups represented by the following formulas:

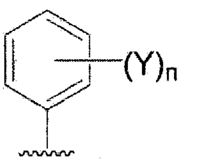 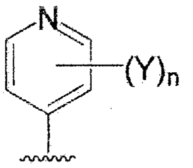 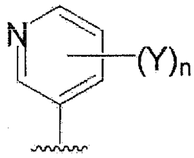 or 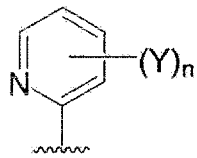

wherein, Y and n are as defined above.

More specific examples thereof include a phenyl group, a 2-halo(for example, 2-F or 2-Cl)phenyl group, a 3-halo(for example, 3-F or 3-Cl)phenyl group, a 4-halo(for example, 4-F or 4-Cl)phenyl group, a 3,5-dihalo (for example, 3,5-di-F or 3,5,di-Cl)phenyl group, a 2,4,6-trihalo(for example, 2,4,6-tri-F or 2,4,6-tri-Cl)phenyl group, a 2,3,5,6-tetrahalo(for example, 2,3,5,6-tetra-F or 2,3,5,6-tetra-Cl)phenyl group, a 1-methoxyphenyl group, a 2-methoxyphenyl group, a 3-methoxyphenyl group, a 2,6-dimethoxyphenyl group, a 1-trifluoromethoxyphenyl group, a 2-trifluoromethoxyphenyl group, and a 3-trifluoromethoxyphenyl group; and a 2-pyridyl group, a 3-pyridyl group, a 4-pyridyl group, a 2-halo(for example, 2-F or 2-Cl)pyridin-4-yl group, a 2,6-dihalo(for example, 2,6-di-F or 2,6-di-Cl)pyridin-4-yl group, a 3-halo(for example, 3-F or 3-Cl)pyridin-2-yl group, and a 2-halo(for example, 2-F or 2-Cl)pyridin-3-yl group.

In the piperidine compound represented by Formula (I), as specific examples of a group represented by the following Formula:

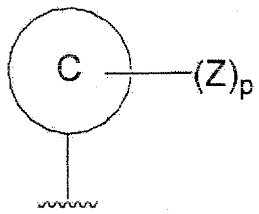

wherein, ring C, Z, and p are as defined above, there are groups represented by the following formulas:

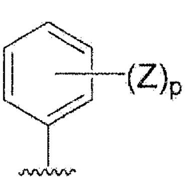 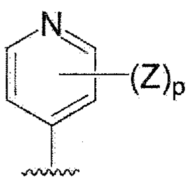 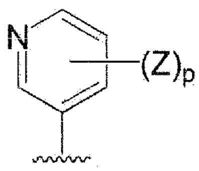 or 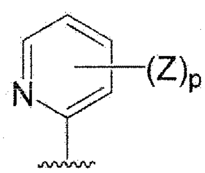

(wherein, Z and p are as defined above).

More specific examples thereof include a phenyl group, a 2-halo(for example, 2-F or 2-Cl)phenyl group, a 3-halo(for example, 3-F or 3-Cl)phenyl group, a 4-halo(for example, 4-F or 4-Cl)phenyl group, a 3,5-dihalo (for example, 3,5-di-F or 3,5,di-Cl)phenyl group, a 2,4,6-trihalo(for example, 2,4,6-tri-F or 2,4,6-tri-Cl)phenyl group, a 2,3,5,6-tetrahalo(for example, 2,3,5,6-tetra-F or 2,3,5,6-tetra-Cl)phenyl group, a 1-methoxyphenyl group, a 2-methoxyphenyl group, a 3-methoxyphenyl group, a 2,6-dimethoxyphenyl group, a 1-trifluoromethoxyphenyl group, a 2-trifluoromethoxyphenyl

group, and a 3-trifluoromethoxyphenyl group; and a 2-pyridyl group, a 3-pyridyl group, a 4-pyridyl group, a 2-halo(for example, 2-F or 2-Cl)pyridin-4-yl group, a 2,6-dihalo (for example, 2,6-di-F or 2,6-di-Cl)pyridin-4-yl group, a 3-halo(for example, 3-F or 3-Cl)pyridin-2-yl group, and a 2-halo(for example, 2-F or 2-Cl)pyridin-3-yl group.

More preferred specific examples of the compound of the present invention include compounds 1 to 54 of the present invention produced in Production Examples 1 to 54 to be described later, and salts thereof.

[Method for producing Compound of Present Invention]

Next, a method for producing the compound of the present invention will be described.

The compound of the present invention can be produce d, for example, in accordance with the following reaction process.

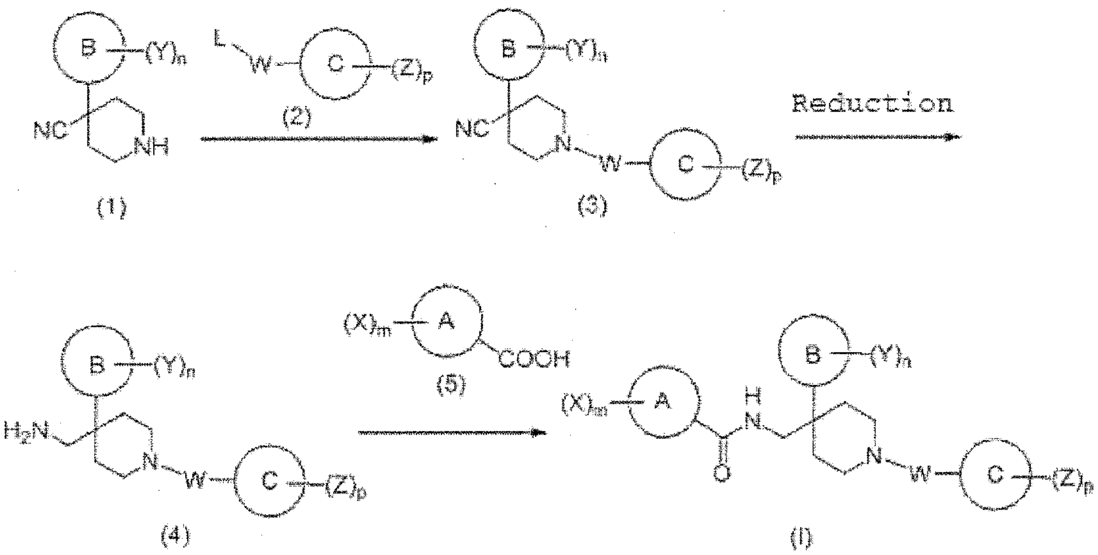


wherein, L is a living group, and ring A, ring B, and Ring C, W, X, Y, Z, m, n, and p are as defined above

The compound represented by Formula (1) can be produ ced in accordance with or based on a well-known method disclosed in Journal of Medicinal Chemistry (2011), 54(13), 4773, for example.

Examples of the leaving group represented by L include a halogen atom (for example, a chlorine atom, a bromine atom, and iodine atom), a methanesulfonyloxy group, a trifluoromethanesulfonyloxy group, a p-toluenesulfonyloxy group, and an o-nitrobenzenesulfonyloxy group.

(1) + (2) → (3) :

The compound represented by Formula (3) can be produ ced by reacting the compound represented by Formula (1) with the compound represented by Formula (2). The compound represented by Formula (1) can be made to have a form of an acid addition salt (for example, hydrochloride) before being subjected to the present reaction.

In general, the present reaction can be performed in a solvent in the presence of a base.

Examples of the base used for the present reaction include inorganic bases, such as sodium hydride, sodium hydroxide, potassium hydroxide, and potassium carbonate; alkali metal alkoxides, such as sodium methoxide, and potassium tert-butoxide; and organic bases, such as triethylamine, 1,4-diazabicyclo[2.2.2]octane, and 1,8-diazabicyclo[5.4.0]-7-undecene.

Examples of the solvent used for the present reaction include ethers, such as diethyl ether, tetrahydrofuran, and dimethoxyethane; acid amides, such as N,N-dimethylformamide; organic sulfur compounds, such as dimethyl sulfoxide and sulfolane; aliphatic hydrocarbons, such as hexane and heptane; aromatic hydrocarbons, such as toluene and xylene; halogenated hydrocarbons, such as 1,2-dichloroethane and chlorobenzene; acetonitrile; water; and mixtures thereof.

In the present reaction, the molar ratio of the compound represented by Formula (2) to the compound represented by Formula (1) to be used can be arbitrarily set. However, the ratio of the compound represented by Formula (2) to 1 mole of the compound represented by Formula (1) is generally 0.5 moles to 10 moles, preferably 0.8 moles to 5 moles, and more preferably 0.9 moles to 2 moles.

The amount of the base used for the present reaction can be generally used at an arbitrary ratio of 0.1 moles to an excessive amount with respect to 1 mole of the compound represented by Formula (1), and preferably 0.5 moles to 3 moles. When the compound represented by Formula (1) forms acid and salt, the the ratio of the amount of the base

is generally 1 mole to an excessive amount, and is preferably 1 mole to 5 moles with respect to 1 mole of the compound represented by Formula (1).

In general, the reaction temperature is within a range of 0°C to 150°C, and in the case of using a solvent of which the boiling point is within this range, the solvent may be refluxed within the temperature range. In general, the reaction time is within a range of 5 minutes to 72 hours.

After completion of the reaction, it is possible to isolate the compound represented by Formula (3) by performing general post-treatment operations, such as organic solvent extraction and condensation, after adding a reaction mixture to water. In addition, the isolated compound represented by Formula (3) can be purified through an operation, such as chromatography, recrystallization, or distillation.

(3) → (4):

The compound represented by Formula (4) can be produ ced by subjecting the compound represented by Formula (3) to a reduction reaction.

In general, the present reaction can be performed in a solvent in the presence of a reducing agent.

Examples of the reducing agent used for the present reaction include lithium aluminum hydride (LAH) ($LiAlH_4$) and borane complex (borane-tetrahydrofuran complex, borane-dimethyl sulfide complex, and diborane).

Examples of the solvent used for the present reaction include ethers, such as diethyl ether, tetrahydrofuran, dimethoxyethane, and dioxane; aliphatic hydrocarbons, such as hexane and heptane; aromatic hydrocarbons, such as toluene and xylene; and mixtures thereof.

The amount of the reducing agent used for the present reaction can be generally used at an arbitrary ratio of 1 mole to an excessive amount with respect to 1 mole of the compound represented by Formula (3), and more preferably 1 mole to 2 moles.

In general, the reaction time is within a range of 5 minutes to 24 hours and the reaction temperature is within a range of -80°C to 50°C.

After completion of the reaction, it is possible to obtain the compound represented by Formula (4) by performing general post-treatment operations, such as organic solvent extraction and condensation, after adding a reaction mixture to water. In addition, the obtained compound represented by Formula (4) can be purified through an operation, such as chromatography, recrystallization, or distillation.

(4) + (5) → (I)

The piperidine compound represented by Formula (I) (compound of the present invention) can be produced by reacting the compound represented by Formula (4) with the compound represented by Formula (5) in the presence of a condensing agent.

In general, the present reaction can be performed in a solvent in the presence of condensing agent, and in the presence of a base as necessary.

Examples of the condensing agent used for the present reaction include dicyclohexyl carbodiimide, diisopropyl carbodiimide, 1-ethyl-3-(3-dimethylaminopropyl) carbodiimide hydrochloride, and benzotriazol-1-yloxy-tris(dimethylamino)phosphonium salt.

Examples of the base used for the present reaction include carbonates, such as sodium carbonate and potassium carbonate; tertiary amines, such as triethylamine, diisopropylethylamine, 1,8-diazabicycl[5.4.0]-7-undecene, and 1,5-diazabicyclo[4.3.0]non-5-ene; and nitrogen-containing aromatic compounds, such as pyridine and 4-dimethylaminopyridine.

Examples of the solvent used for the present reaction include aliphatic hydrocarbons, such as benzene, toluene, and hexane; ethers, such as diethyl ether, tetrahydrofuran, 1,4-dioxane, and tert-butyl methyl ether; halogenated hydrocarbons, such as dichloromethane, chloroform, 1,2-dichloroethane, and chlorobenzene; acid amides, such as N,N-dimethylformamide; and mixtures thereof.

In the present reaction, the molar ratio of the compound represented by Formula (5) to the compound represented by Formula (4) to be used can be arbitrarily set. However, the ratio of the compound represented by Formula (4) to 1 mole of the compound represented by Formula (5) is generally 0.5 moles to 5 moles, preferably 0.8 moles to 3 moles, and more preferably 0.9 moles to 1.5 moles.

The amount of the condensing agent used for the present reaction can be generally used at an arbitrary ratio of 1 mole to an excessive amount with respect to 1 mole of the compound represented by Formula (5), and preferably 1 mole to 3 moles.

In a case of using a base for the present reaction, the amount of the base can be generally used at an arbitrary ratio of 1 mole to an excessive amount with respect to 1 mole of the compound represented by Formula (5), and preferably 1 mole to 3 moles.

In general, the reaction time is within a range of 5 minutes to 72 hours and the reaction temperature is within a range of -20°C to 100°C.

After completion of the reaction, it is possible to isolate the compound of the present invention by performing general post-treatment operations, such as organic solvent extraction and condensation, after adding a reaction mixture

to water. In addition, the isolated compound of the present invention can be purified through an operation, such as chromatography, recrystallization, or distillation.

The raw material compound and the reaction intermediate compounds (compounds represented by Formula (1) to Formula (5)) used in the above-described reaction process can protect a functional group using an appropriate protective group through a well-known method as necessary before being provided to the reaction, and can deprotect the protective group through a well-known method after the completion of the reaction. In addition, it is possible to use a starting material compound and a target compound in an appropriate salt form.

In the compound of the present invention, there is a compound which can form acid addition salts, such as hydrochloride, nitrate, sulfate, hydrobromide, phosphate, carbonate, acetate, lactate, and citrate.

[Application for Controlling Pests]

Examples of pests for which the compound of the present invention has an effect include harmful arthropods, such as harmful insects or harmful mites; nematodes; and mollusks. Specific examples of the pests include the following.

Hemiptera pests: planthoppers, such as small brown planthoppers (Laodelphax striatellus), brown planthoppers (Nilaparvata lugens), and white-backed planthoppers (Sogatella furcifera); leafhoppers, such as green rice leafhoppers (Nephotettix cincticeps), Taiwan green rice leafhoppers (Nephotettix virescens), and tea green leafhoppers (Empoasca onukii); aphids, such as cotton aphids (Aphis gossypii), green peach aphids (Myzus persicae), cabbage aphids (Brevicoryne brassicae), spiraea aphids (Aphis spiraecola), potato aphids (Macrosiphum euphorbiae), greenhouse potato aphids (Aulacorthum solani), bird-cherry oat aphids (Rhopalosiphum aphids padi), black citrus aphids (Toxoptera citricidus), and mealy plum aphids (Hyalopterus pruni); stink bugs, such as eastern green stink bugs (Nezara antennata), bean bugs (Riptortus clavetus), male rice bugs (Leptocorisa chinensis), white-spotted spined bugs (Eysarcoris parvus), and brown marmorated stink bugs (Halyomorpha mista); whiteflies, such as greenhouse whiteflies (Trialeurodes vaporariorum), silverleaf whiteflies (Bemisia tabaci), citrus whiteflies (Dialeurodes citri), and orange spiny whiteflies (Aleurocanthus spiniferus); scale insects, such as red scales (Aonidiella aurantii), San Jose scales (Comstockaspis perniciosa), citrus snow scales (Unaspis citri), red wax scales (Ceroplastes rubens), cottony cushion scales (Icerya purchasi), mealybugs (Planococcus kraunhiae), comstock mealybugs (Pseudococcus longispinis), and white peach scales (Pseudaulacaspis pentagona); lace bugs; bedbugs, such as Cimex lectularius; psyllids; and the like.

Lepidoptera pests: pyralids, such as asiatic rice borers (Chilo suppressalis), yellow stem borers (Tryporyza incertulas), rice leafrollers (Cnaphalocrocis medinalis), cotton leaf rollers (Notarcha derogata), Indian meal moths (Plodia interpunctella), Asian corn borers (Ostrinia furnacalis), cabbage webworms (Hellula undalis), and bluegrass webworms (Pediasia teterrellus); noctuids, such as oriental leafworm moths (Spodoptera litura), beet armyworms (Spodoptera exigua), oriental armyworms (Pseudaletia separata), cabbage moths (Mamestra brassicae), black cutworms (Agrotis ipsilon), asiatic common loopers (Plusia nigrisigna), the genus Trichoplusia, the genus Heliothis, and the genus Helicoverpa; pieridae, such as small white butterflies (Pieris rapae); leaf roller moths, such as the genus Adoxophyes, oriental fruit moths (Grapholita molesta), soybean pod borers (Leguminivora glycinivorella), adzuki bean podworms (Matsumuraeses azukivora), summer fruit tortrix moths (Adoxophyes orana fasciata), smaller tea tortrixes (Adoxophyes honmai.), oriental tea tortrix moths (Homona magnanima), apple tortrixes (Archips fuscocupreanus), and codling moths (Cydia pomonella); meaf miners, such as tea leaf rollers (Caloptilia theivora), and apple leaf miner (Phyllonorycter ringoneella); codling moths, such as peach fruit moths (Carposina niponensis); leafminer moths, such as the genus Rionetia; tussock moths, such as the genus Lymantria and the genus Euproctis; ermine moths, such as diamondback moths (Plutella xylostella); pink bollworms (Pectinophora gossypiella) and potato tuber moths (Phthorimaea operculella); tiger moths, such as fall webworms (Hyphantria cunea); and tineids, such as clothes moths (Tinea translucens), common clothes moths (Tineola bisselliella); and the like.

Thysanoptera pests: trips, such as western flower thrips (Frankliniella occidentalis), southern yellow thrips (Thrips parmi), yellow tea thrips (Scirtothrips dorsalis), onion thrips (Thrips tabaci), and flower thrips (Frankliniella intonsa); and the like.

Diptera pests: house mosquitoes, such as common house mosquitoes (Culex pipiens pallens), culex mosquitoes (Culex tritaeniorhynchus), and southern house mosquitoes (Culex quinquefasciatus); stegomyia, such as tallow fever mosquitoes (Aedes aegypti) and tiger mosquitoes (Aedes albopictus); anopheles, such as hyrcanus group mosquitoes (Anopheles sinensis) and gambiae sensu strictoes (Anopheles gambiae); midges; house flies, such as common houseflies (Musca domestica) and false stable flies (Muscina stabulans); greenbottle flies; fleshflies; Fannia canicularis; rootmaggots, such as bean seed flies (Delia platura) and onion flies (Delia antiqua); leafminer flies, such as Japanese rice leafminers (Agromyza oryzae), rice leafminers (Hydrellia griseola), vegetable leafminers, (Liriomyza sativae), American serpentine leafminers (Liriomyza trifolii), and garden pea leafminers (Chromatomyia horticola); fruit flies, such as rice stem maggots (Chlorops oryzae); fruit flies, such as melon flies (Dacus cucurbitae) and Mediterranean fruit flies (Ceratitis capitata); dorsophilas; phorid flies, such as flea flies (Megaselia spiracularis); sand flies, such as moth flies (Clogmia albipunctata); fungus gnats; buffalo gnats; horse flies, such as gadflies (Tabanus trigonus); louse flies; stable flies; and

the like.

Elytron pests: corn rootworms, such as western corn rootworms (Diabrotica virgifera virgifera) and southern corn rootworms (Diabrotica undecimpunctata howardi); scarabaeid beetles, such as scarab beetles (Anomala cuprea), soybean beetles (Anomala rufocuprea), and Japanese beetles (Popillia japonica); weevils, such as maize weevils (Sitophilus zeamais), rice water weevils (Lissorhoptrus oryzophilus), adzuki bean weevils (Callosobruchuys chienensis), rice plant weevils (Echinocnemus squameus), boll weevils (Anthonomus grandis), and hunting billbugs (Sphenophorus venatus); mealworms, such as tallow mealworms (Tenebrio molitor) and red flour beetles (Tribolium castaneum); leaf beetles, such as rice leaf beetles (Oulema oryzae), cucurbit leaf beetles (Aulacophora femoralis), striped flea beetles (Phyllotreta striolata), and Colorado potato beetles (Leptinotarsa decemlineata); carpet beetles, such as varied carpet beetles (Anthrenus verbasci), and hide beetles (Dermestes maculates); deathwatch beetles, such as cigarette beetles (Lasioderma serricorne); ladybugs, such as 28-spotted ladybirds (Epilachna vigintioctopunctata); bark beetles, such as powderpost beetles (Lyctus brunneus) and common pine shoot beetles (Tomicus piniperda); auger beetles; museum beetles; sawyer beetles, such as citrus long-horned beetles (Anoplophora malasiaca); click beetles (Agriotes spp.), and rove beetles (Paederus fuscipes); and the like.

Orthopteran pests: migratory locusts (Locusta migratoria), mole crickets (Gryllotalpa africana), rice grasshoppers (Oxya yezoensis), rice grasshoppers (Oxya japonica), crickets, and the like.

Siphonaptera pests: cat fleas (Ctenocephalides felis), dog fleas (Ctenocephalides canis), human fleas (Pulex irritans), oriental rat fleas (Xenopsylla cheopis), and the like.

Anoplura pests: body lice (Pediculus humanus corporis), head lice (Pediculus humanus humans), pubic lice (Phthirus pubis), short-nosed cattle lice (Haematopinus eurysternus), pig lice (Haematopinus suis), dog sucking lice (Linognathus setosus), and the like.

Mallophaga pests: sheep lice (Dalmalinia ovis), biting lice(Dalmalinia bovis), chicken shaft lice (Menopon gallinae), canine chewing lice (Trichodectes canis), cleaning agent chewing lice (Felicola subrostrata), and the like.

Hymenoptera pests: ants, such as pharaoh ants (Monomorium pharaosis), Japanese wood ants (Formica fusca japonica), black house ants (Ochetellus glaber), queenless ants (Pristomyrmex pungens), big-headed ants (Pheidole noda), leafcutter ants (Acromyrmex spp.), and fire ants (Solenopsis spp.); wasps; bethylid wasps; sawflies, such as turnip sawflies (Athalia rosae), cabbage sawflies (Athalia japonica); and the like.

Blattodea Pests: German cockroaches (Blattella germanica), smoky brown cockroaches (Periplaneta fuliginosa), American cockroaches (Periplaneta americana), brown cockroaches (Periplaneta brunnea), oriental cockroaches (Blatta orientalis), and the like.

Isoptera Pests: Yamato termites (Reticulitermes speratus), Formosan subterranean termites (Coptotermes formosanus), western drywood termites (Incisitermes minor), Daikoku drywood termites (Cryptotermes domesticus), Taiwan termites (Odontotermes formosanus), dry-wood termites (Neotermes koshunensis), red tree termites (Glyptotermes satsumensis), Nakajima termites (Glyptotermes nakajimai), black tree termites (Glyptotermes fuscus), Kodama termites (Glyptotermes kodamai), Kushimoto termites (Glyptotermes kushimensis), Japanese damp-wood termites (Hodotermopsis japonica), Koshuie termites (Coptotermes guangzhoensis), Amami termites (Reticulitermes miyatakei), yellow limb termites (Reticulitermes flaviceps amamianus), Kanmon termites (Reticulitermes sp.), wood-eating higher termites (Nasutitermes takasagoensis), Nitobe termites (Pericapritermes nitobei), Musha termites (Sinocapritermes mushae), Reticuliterumes flavipes, Reticulitermes hesperus, Reticulitermes virginicus, Reticulitermes tibialis, Heterotermes aureus, Zootermopsis nevadensis, and the like.

Acarina pests: spider mites, such as two-spotted spider mites (Tetranychus urticae), kanzawai spider mites (Tetranychus kanzawai), citrus red mites (Panonychus citri), European red mites (Panonychus ulmi), and the genus Origonikasu; eriophyidae, such as Mandarin orange rust mites (Aculops pelekassi), pink citrus rust mites (Phyllocoptruta citri), tomato rust mites (Aculops lycopersici), purple mites (Calacarus carinatus), tea rust mites (Acaphylla theavagrans), false pear rust mites (Eriophyes chibaensis), and apple rust mites (Aculus schlechtendali); white mites, such as broad mites (Polyphagotarsonemus latus); false spider mites, such as red and black flat mites (Brevipalpus phoenicis); peacock mites; ticks, such as three-host ticks (Haemaphysalis longicornis), yellow ticks (Haemaphysalis flava), Taiwan leather ticks (Dermacentor taiwanicus), American dog ticks (Dermacentor variabilis), ovate hard ticks (Ixodes ovatus), taiga ticks (Ixodes persulcatus), blackleged ticks (Ixodes scapularis), lone star ticks (Amblyomma americanum), cattle ticks (Boophilus microplus), and brown dog ticks (Rhipicephalus sanguineus); flour mites, such as mould mites (Tyrophagus putrescentiae) and acarid mites (Tyrophagus similis); acariform mites, such as American house dust mites (Dermatophagoides farinae) and European house dust mites (Dermatophagoides ptrenyssnus); cheyletid mites, such as hunting mites (Cheyletus eruditus), Malacca meat mites (Cheyletus malaccensis), Minami Tsumedani (Cheyletus moorei), and dog mites (Cheyletiella yasguri); itch mites, such as ear mites (Octodectes cynotis) and itch mites (Sacroptes scabiei); follicle mites, such as follicle mites (Demodex canis); Listrophorid mites; oribatid mites; parasitoid mites, such as tropical rat mites (Ornithonyssus bacoti), northern fowl mites (Ornithonyssus sylvairum), and chicken mites (Dermanyssus gallinae); trombiculid mites, such as red fiber sand mites (Leptotrombidium akamushi); arachnids, such as Japanese foliage spiders (Chiracanthium japonicum) and redback spiders (Latrodectus hasseltii); and the like.

Chilopoda: house centipedes (Thereuonema hilgendorfi), Chinese red-headed centipedes (Scolopendra subspinipes), and the like.

Diplopoda: garden millipedes (Oxidus gracilis), red millipedes (Nedyopus tambanus), and the like.

Isopoda: pill-bugs (Armadillidium vulgare), and the like.

Gastropoda: air-breathing land slugs (Limax marginatus), yellow slugs (Limax flavus), and the like.

Nematodes: rice white tip nematodes (Aphelenchoides besseyi), strawberry bud nematodes (Nothotylenchus acris), southern root-knot nematodes (Meloidogyne incognita), northern root-knot nematodes (Meloidogyne hapla), Javanese root-knot nematodes (Meloidogyne javanica), soybean cyst nematodes (Heterodera glycines), potato cyst nematodes (Globodera rostochiensis), South meadow nematodes (Pratylenchus coffeae), wheat meadow nematodes (Pratylenchus neglectus), and the like.

A pest control agent of the present invention compri ses the compound of the present invention and an inert carrier. In general, the pest control agent of the present invention has been formulated into an emulsion, an oil solution, powder, granules, a water-dispersible agent, a flowable agent, a microcapsule agent, an aerosol agent, a smoking agent, a poison bait agent, a resin formulation, etc. by mixing the the compound of the present invention with an inert carrier, such as a solid carrier, liquid carrier, and gaseous carrier, and, as necessary, adding a surfactant and other auxiliary agents for a formulation.

In general, the pest control agent of the present invention contains 0.01 wt% to 95 wt% of the compound of the present invention.

Examples of the solid carrier used during the formulation include fine powder and particulates, such as clays (kaolin clay, diatomaceous earth, bentonite, Fubasami clay, acid clay, and the like), synthetic hydrated silicon oxide, talc, ceramics, other inorganic minerals (sericite, quartz, sulfur, activated carbon, calcium carbonate, hydrated silica, and the like), and chemical fertilizers (ammonium sulfate, ammonium phosphate depreciation, ammonium nitrate, urea, ammonium chloride, and the like).

Examples of the liquid carrier include water, alcohols (methanol, ethanol, isopropyl alcohol, butanol, hexanol, benzyl alcohol, ethylene glycol, propylene glycol, phenoxyethanol, and the like), ketones (acetone, methyl ethyl ketone, cyclohexanone, and the like), aromatic hydrocarbons (toluene, xylene, ethylbenzene, dodecylbenzene, phenylxylylethane, methylnaphthalene, and the like), aliphatic hydrocarbons (hexane, cyclohexane, kerosine, gas oil, and the like), esters (ethyl acetate, butyl acetate, isopropyl myristate, ethyl oleate, diisopropyl adipate, diisobutyl adipate, propylene glycol monomethyl ether acetate, and the like), nitriles (acetonitrile, isobutyronitrile, and the like), ethers (diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether, 3-methoxy-3-methyl-1-butanol), acid amides (N,N-dimethylformamide, N,N-dimethylacetamide, and the like), halogenated hydrocarbons (dichloromethane, trichloroethane, carbon tetrachloride, and the like), sulfoxides (dimethyl sulfoxide, etc.), propylene carbonate, and vegetable oils (soybean oil, cottonseed oil, and the like).

Examples of the gaseous carrier include fluorocarbon, butane gas, LPG (liquefied petroleum gas), dimethyl ether, and carbon dioxide.

Examples of the surfactant include non-ionic surfactants, such as polyoxyethylene alkyl ether, polyoxyethylene alkylaryl ether, and polyethylene glycol fatty acid ester; and anionic surfactants, such as alkyl sulfonate, alkyl benzene sulfonate, and alkyl sulfate.

Examples of the auxiliary agent for a formulation include a sticking agent, a dispersing agent, a stabilizer and a colorant, and specific examples thereof include casein, gelatin, sugars (starch, gum arabic, cellulose derivatives, alginic acid, and the like), lignin derivatives, bentonite, synthetic water-soluble polymers (polyvinyl alcohol, polyvinyl pyrrolidone, polyacrylic acids, and the like), PAP (isopropyl acid phosphate), BHT (2,6-di-tert-butyl-4-methylphenol), and BHA (a mixture of 2-tert-butyl-4-methoxyphenol and 3-tert-butyl-4-methoxyphenol).

A method for controlling pests in the present invention is performed by directly applying an effective amount of the compound of the present invention to pests and/or applying an effective amount of the compound of the present invention to a habitat (plant bodies, soil, the inside of a house, animal bodies, and the like) of the pests. In the method for controlling pests of the present invention, the compound of the present invention is generally used in the form of a pest control agent of the present invention.

In a case of using the pest control agent of the present invention for controlling pests in agricultural fields, the application amount thereof is 1 g to 10000 g of the compound of the present invention per 10000 $m^2$. When the pest control agent of the present invention has been formulated into an emulsion, a water-dispersible agent, a flowable agent, etc., in general, the compound of the present invention is applied by being diluted in water so that the concentration of the compound of the present invention becomes 0.01 ppm to 10000 ppm. Granules, powder, etc. are generally applied to the subject as they are.

These formulations or water-diluted solutions of the formulations may be sprayed directly onto pests or onto plants, such as crops, to be protected from the pests. Alternately, soil in cultivated land may be treated with the formulations or the water-diluted solutions in order to control pests which live in the soil.

In addition, the treatment can be performed through methods, such as winding of a resin formulation which has been processed into a sheet shape or a string shape onto crops, stretching the resin formulation across the vicinity of the crops, and spreading the resin formulation onto soil near the roots.

In the case of using the pest control agent of the present invention for controlling pests which live inside of a house, in general, the application amount thereof is 0.01 mg to 1000 mg of the compound of the present invention per 1 $m^2$ of the treatment area when being treated on a surface, and is 0.01 mg to 500 mg of the compound of the present invention per 1 $m^3$ of the treatment space when being treated in a space. When the pest control agent of the present invention has been formulated into an emulsion, a wettable agent, a flowable agent, etc, in general, the compound of the present invention is applied by being diluted in water so that the concentration of the compound of the present invention becomes 0.1 ppm to 1000 ppm. An oil solution, an aerosol agent, a smoking agent, a poison bait agent, etc. are generally applied to the subject as they are.

The pest control agent of the present invention can be used in agricultural land in which the following "crops" have been cultivated.

Farm products: corn, rice, wheat, barley, rye, oats, sorghum, cotton, soybeans, peanuts, buckwheat, sugar beets, rapeseeds, sunflowers, sugar cane, tobacco, and the like.

Vegetables: solanaceous vegetables (eggplants, tomatoes, bell peppers, peppers, potatoes, and the like), cucurbit vegetables (cucumbers, pumpkins, zucchinis, watermelons, melons, and the like), cruciferous vegetables (radishes, turnips, horseradishes, kohlrabi, chinese cabbage, cabbage, mustard, broccoli, cauliflower, and the like), asteraceae vegetables (burdock, garland chrysanthemum, artichoke, lettuce, and the like), liliaceae vegetables (spring onions, onions, garlic, asparagus), umbelliferae vegetables (carrots, parsley, celery, parsnips, and the like), chenopodiaceae vegetables (spinach, chard, and the like), labiatae vegetables (perilla, mint, basil, and the like), strawberries, sweet potatoes, Japanese yams, taro, and the like.

Fruit trees: pomaceous fruits (apples, European pears, Japanese pears, Chinese quinces, quinces, and the like), stone fruits (peaches, Japanese plums, nectarines, Chinese plums, cherries, apricots, prunes, and the like), citrus (satsuma mandarins, oranges, lemons, limes, grapefruits, and the like), nut trees (chestnut, walnut, hazelnut, almond, pistachio, cashew nut, macadamia nut, and the like), berry fruits (blueberries, cranberries, blackberries, raspberries, and the like), grapes, oysters, olives, loquats, bananas, coffee, date palms, coconuts, oil palms, and the like.

Trees other than fruit trees: tea, mulberry, flowering trees (azalea, camellia, hydrangea, sasanqua, Japanese star anise, cherry, tulip tree, crape myrtle, fragrant olive, and the like), street trees (ash, birch tree, dogwood, eucalyptus, ginkgo, lilac, maple, oak, poplar, cercis, sweetgum, sycamore, Japanese zelkova, Japanese arborvitae, fir tree, hemlock, juniper, pine, spruce, yew, elm, horse chestnut, and the like), sweet viburnum, podocarpus, cedar, Japanese cypress, croton, Japanese spindle tree, Japanese photinia, and the like.

Lawn: grasses (zoysiagrass, zoysia matrella, and the like), bermuda grasses (bermuda grass, and the like), bent grasses (creeping bent, creeping bent grass high, thread creeping bent grass, and the like), blue grasses (kentucky bluegrass, rough bluegrass, and the like), fescues (festuca arundinacea, chewings fescue, creeping red fescue, and the like), ryegrasses (darnel, rye grass, and the like), orchard grass, timothy grass, and the like.

Others: flowering plants (rose, carnation, chrysanthemum, lisianthus, gypsophila, gerbera, marigold, salvia, petunia, verbena, tulip, aster, gentian, lily, pansies, cyclamen, orchid, lily of the valley, lavender, stock, ornamental cabbage, primula, poinsettia, gladiolus, cattleya, daisy, cymbidium, begonia, and the like), biofuel plants (jatropha, curcas, safflower, camelina, switchgrass, miscanthus, reed canarygrass, giant reed, kenaf, cassava, willow, algae, and the like), foliage plants, and the like.

Genetically modified crops are also included in "crops".

It is possible to use the pest control agent of the present invention by mixing with or together with other insecticides, acaricides, nematicides, fungicides, plant growth regulators, herbicides, and synergists. Examples of active ingredients of the insecticides, the acaricides, the nematicides, the fungicides, the herbicides, and the synergists are shown as follows.

Active Ingredients of Insecticides

(1) Carbamate-based Compounds

Alanycarb, aldicarb, bendiocarb, benfuracarb, butocarboxim, butoxycarboxim, carbaryl (=NAC), carbofuran, carbosulfan, cloethocarb, ethiofencarb, fenobucarb (=BPMC), fenothiocarb, formetanate, furathiocarb, isoprocarb (=MIPC), methiocarb, methomyl, metolcarb, oxamyl, pirimicarb, propoxur (:PHC), thiodicarb, thiofanox, triazamate, trimethacarb, XMC, and xylylcarb.

(2) Organic Phosphorus Compounds

Acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos (=CYAP), demeton-S-methyl, diazinon, dichlorvos (=DDVP), dicrotophos, dimethoate, dimethylvinphos, disulfoton, EPN, ethion, ethoprophos, etrimfos, famphur, fenamiphos, fenitrothion (=MEP), fenthion (=MPP), fosthiazate, heptenophos, imicyfos, isofenphos, isopropyl O-(methoxyaminothio-phosphoryl)salicylate, isoxathion, malathion, mecarbam, mesulfenfos, methamidophos, methidathion (=DMTP), mevinphos, monocrotophos, naled (:BRP), omethoate, oxydemeton-methyl, parathion, parathion-methyl, phenthoate (=PAP), phorate, phosalone, phosmet (=PMP), phosphamidon, phoxim, pirimiphos-methyl, profenofos, propetamphos, prothiofos, pyraclorfos, pyridafenthion, quinalphos, sulfotep, tebupirimfos, temephos, terbufos, tetrachlorvinphos, thiometon, triazophos, trichlorphon (=DEP), vamidothion, aluminiumphosphide, butathiofos, dichlorodiisopropylether (DCIP), dichlofenthion (=ECP), formothion, hydrogenphosphide, oxydeprofos (=ESP), propaphos, salithion, bromphenophos, and sulprofos.

(3) Pyrethroid Compounds

Acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, gamma-cyhalothrin, furamethrin, tau-fluvalinate, metofluthrin, profluthrin, dimefluthrin, protrifenbute, 2,3,5,6-tetrafluoro-4-(methoxymethyl)benzyl(EZ)-(1RS,3RS;1RS,3SR)-2,2-dimethyl-3-prop-1-enyl-cyclopropanecarboxylate, 2,3,5,6-tetrafluoro-4-methyl-benzyl(EZ)-(1RS,3RS;1RS,3SR)-2,2-dimethyl-3-prop-1-enyl-cyclopropanecarboxylate, 2,3,5,6-tetrafluoro-4-(methoxymethyl)benzyl(1RS,3RS;1RS,3SR)-2,2-dimethyl-3-(2-methyl-1-propenyl)cyclopropanecarboxylate, and the like.

(4) Nereistoxin Compounds

Cartap, bensultap, thiocyclam, thisultap, monosultap, and bisultap.

(5) Neonicotinoid Compounds

Imidacloprid, nitenpyram, acetamiprid, thiamethoxam, thiacloprid, dinotefuran, and clothianidin.

(6) Benzoylurea Compounds

Bistrifluron, chlorfluazuron, diflubenzuron, flucycloxuron, flufenoxuron, hexaflumuron, lufenuron, novaluron, noviflumuron, teflubenzuron, triflumuron, and fluazuron.

(7) Phenylpyrazole Compounds

Acetoprole, ethiprole, fipronil, vaniliprole, pyriprole, and pyrafluprole.

(8) Bt Toxin Insecticides

Viable spores derived from Bacillus thuringiensis bacteria and crystal toxins produced from Bacillus thuringiensis bacteria, and a mixture thereof.

(9) Hydrazine Compounds

Chromafenozide, halofenozide, methoxyfenozide, and tebufenozide.

(10) Organic Chlorine Compounds

Aldrin, dieldrin, dienochlor, endosulfan, methoxychlor, chlordane, and DDT.

(11) Other Ingredients of Insecticides

Machine oil, nicotine-sulfate, nicotine, abamectin, emamectin-benzoate, milbemectin, lepimectin, spinosad, spinetoram, indoxacarb, metaflumizone, amidoflumet, metoxadiazone, buprofezin, cyromazine, hydroprene, methoprene, kinoprene, pyriproxyfen, fenoxycarb, chlorphenapyr, DNOC, diafenthiuron, pymetrozine, flonicamid, pyrifluquinazone, pyridalyl, tolfenpyrad, flurimfen, flufenerim, spirotetramat, spiromesifen, sulfoxaflor, chlorantraniliprole, cyantraniliprole, flubendiamide, a compound represented by the following Formula (K):

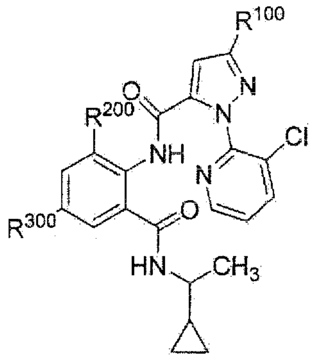

(K)

(wherein, $R^{100}$ represents a chlorine atom, a bromine atom, or a trifluoromethyl group, $R^{200}$ represents a chlorine atom, a bromine atom or a methyl group, and $R^{300}$ represents a chlorine atom, a bromine atom or a cyano group), a compound represented by the following Formula (L):

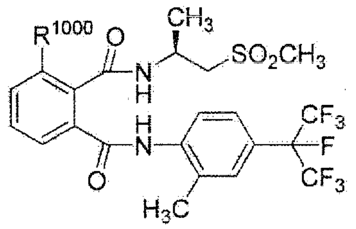

(L)

(wherein, $R^{1000}$ represents a chlorine atom, a bromine atom, or an iodine atom), metoxadiazone, sulfluramid, arsenic acid, benclothiaz, lime nitrogen (calcium cyanamide), lime sulfur (calcium polysulfide), metam-ammonium, metam-sodium, potassium oleate, sulfur, and tralopyril.

Active Ingredients of Acaricides

Acequinocyl, amitraz, benzoximate, bifenaate, bromopropylate, chinomethionat, chlorobenzilate, chlorfenson (CPCBS), clofentezine, cyflumetofen, dicofol, etoxazole, fenazaquin, fenbutatin oxide, fenothiocarb, fenpyroximate, fluacrypyrim, hexythiazox, propargite (=BPPS), polynactins, pyridaben, pyrimidifen, tebufenpyrad, tetradifon, spirodiclofen, amidoflumet, and cyenopyrafen.

Active Ingredients of Nematicides

Methyl bromide, D-D 1,3-dichloropropene, DCIP, fosthiazate, levamisole hydrochloride (levamisol), methyisothiocyanate, piperazine citrate, tribromsalan, nitroxynil, triclabendazole, oxyclozanide, bithionol, tartaric acid morantel (morantel tartarate), pyrantel pamoate, praziquantel, derquantel, febantel, oxantel, closantel, clorsulon, emodepside, nitroscanate, albendazole, fenbendazole, flubendazole, parbendazole, thiabendazole, cambendazole, mebendazole, destomycin A (destomycin), hygromycin B (hygromycin), milbemycin, ivermectin, doramectin, moxidectin, eprinomectin, selamectin, diethylcarbamazine citrate, dithiazanine iodide, melarsomine, disophenol, methyridine, santonin, and imicyafos.

Active Ingredients.

Sterol biosynthesis-inhibiting compounds, such as azaconazole, bitertanol, bromuconazole, cyproconazole,

difenoconazole, diniconazole-M, epoxiconazole, fenarimol, fenbuconazole, flusilazole, fluquinconazole, flutriafol, hexaconazole, imazalil, imibenconazole, ipconazole, metconazole, myclobutanil, nuarimol, oxpoconazole, pefurazoate, penconazole, prochloraz, propiconazole, prothioconazole, pyrifenox, simeconazole, tebuconazole, tetraconazole, itraconazole, fluconazole, miconazole, ketoconazole, voriconazole, triadimefon, triadimenol, triflumizole, trifolin, triticonazole, aldimorph, dodemorph, fenpropimorph, tridemorph, fenpropidin, piperalin, spiroxamine, fenhexamide, pyributicarb, naphtifine, terbinafine, nystatin, clotrimazole, econazole, isoconazole, sulconazole, oxiconazole, bifonazole, neticonazole, lanoconazole, and luliconazole;
cytostatic fungicidal compounds, such as benomyl, carbendezim, fuberidazole, thiabendazole, thiophanate, thiphanate, thiophanate-methyl, diethofencarb, zoxamide, pencycuron, and fluopicolide;
signal transmission system-inhibiting fungicidal compounds, such as chlozolinate, iprodione, procymidone, vinclozolin, quinoxyfen, proquinazid, fenpiclonil, and fludioxonil;
amino acid biosynthesis-inhibiting fungicidal compounds, such as cyprodinil, pyrimethanil, and mepanipyrim;
nucleic acid biosynthesis-inhibiting fungicidal compounds, such as metalaxyl, benalaxyl, and oxolinic acid;
lipid-membrane systhesis-inhibiting fungicidal compounds, such as isoprothiolane, iprobenfos, tolclofos-methyl, prothiocarb, dimethomorph, benthiavalicarb-isopropyl, iprovalicarb, and mandipropamid;
melanin biosynthesis-inhibiting fungicidal compounds, such as diclocymet, carpropamid, fenoxanil, fthalide, tricyclazole, and pyroquilon;
strobilurin fungicidal compounds, such as azoxystrobin, dimoxystrobin, enestrobin, fluoxastrobin, kresoxim-methyl, metominostrobin, orysastrobin, picoxystrobin, pyraclostrobin, pyribencarb, and trifloxystrobin;
anilide fungicidal compounds, such as carboxin, oxycarboxin, mepronil, flutoranil, furametopyr, thifluzamide, boscalid, penthiopyrad, fluopyram, bixafen, isopyrazam, penflufen, sedaxan, and fluxapyroxad;
resistance-inducing compounds, such as acibenzolar-S-methyl, probenazole, tiadinil, and isotianil;
glucan synthesis-inhibiting fungicidal compounds, such as validamycin A and polyoxin B;
protein biosynthesis-inhibiting fungicidal compounds, such as blastcidin-S, kasugamycin, and streptomycin;
fungicidal compounds of multi-site contact activity, such as thiuram, mancozeb, chlorothalonil, captan, dichlofluanid, folpet, iminoctadine, and ziram;
amisulbrom; cyazofamid; ferimzone; fluazinam; binapacryl; dinocap; famoxadone; fenamidone; ethaboxam; metrafenone; cyflufenamid, amphotericin B, flucytosine, etc. Active Ingredients of Herbicides

(1) Phenoxyfatty Acid Herbicidal Compounds

2,4-PA, MCP, MCPB, phenothiol, mecoprop, fluroxypyr, triclopyr, clomeprop, naproanilide.

(2) Benzoic Acid Herbicidal Compounds

2,3,6-TBA, dicamba, clopyralid, picloram, aminopyralid, quinclorac, and quinmerac.

(3) Urea Herbicidal Compounds

Diuron, linuron, chlortoluron, isoproturon, fluometuron, isouron, tebuthiuron, methabenzthiazuron, cumyluron, daimuron, and methyl-daimuron.

(4) Triazine Herbicidal Compounds

Atrazine, ametoryn, cyanazine, simazine, propazine, simetryn, dimethametryn, prometryn, metribuzin, triaziflam, and indaziflam.

(5) Bipyridinium Herbicidal Compounds

Paraquat and diquat.

(6) Hydroxy Benzonitrile Herbicidal Compounds

Bromoxynil and ioxynil.

(7) Dinitroaniline Herbicidal Compounds

Pendimethalin, prodiamine, and trifluralin.

(8) Organic Phosphorus Herbicidal Compounds

Amiprofos-methyl, butamifos, bensulide, piperophos, anilofos, glyphosate, glufosinate, glufosinate-P, and bialaphos.

(9) Carbamate Herbicidal Compounds

Di-allate, tri-allate, EPTC, butylate, benthiocarb, esprocarb, molinate, dimepiperate, swep, chlorpropham, chlorpropham, phenmedipham, phenisopham, pyributicarb, and asulam.

(10) Acid Amide Herbicidal Compounds

Propanil, propyzamide, bromobutide, and etobenzanid.

(11) Chloroacetanilide Herbicidal Compounds

Acetochlor, alachlor, butachlor, dimethenamid, propachlor, metazachlor, metolachlor, pretilachlor, thenylchlor, and pethoxamid.

(12) Diphenyl Ether Herbicidal Compounds

Acifluorfen (acifluorfen-sodium), bifenox, oxyfluorfen, lactofen, fomesafen, chlomethoxynil, and aclonifen.

(13) Cyclic Imide Herbicidal Compounds

Oxadiazon, cinidon-ethyl, carfentrazone-ethyl, surfentrazone, flumiclorac-pentyl, flumioxazin, pyraflufen-ethyl, oxadiargyl, pentoxazone, fluthiacet-methyl, butafenacil, benzfendizone, bencarbazone, and saflufenacil.

(14) Pyrazole Herbicidal Compounds

Benzofenap, pyrazolate, pyrazoxyfen, topramezone, and pyrasulfotole.

(15) Triketone Herbicidal Compounds

Isoxaflutole, benzobicyclon, sulcotrione, mesotrione, tembotrione, and tefuryltrione.

(16) Aryloxyphenoxypropionic Acid Herbicidal Compounds

Clodinafop-propargyl, cyhalofop-butyl, diclofop-methyl, fenoxaprop-ethyl, fluazifop-butyl, haloxyfop-methyl, quizalofop-ethyl, and metamifop.

(17) Trione Oxime Herbicidal Compounds

Alloxydim (alloxydim-sodium), sethoxydim, butroxydim, clethodim, cloproxydim, cycloxydim, tepraloxydim, tralkoxydim, and profoxydim.

(18) Sulfonylurea Herbicidal Compounds

Chlorsulfuron, sulfometuron-methyl, metsulfuron-methyl, chlorimuron-ethyl, tribenuron-methyl, triasulfuron, bensulfuron-methyl, thifensulfuron-methyl, pyrazosulfuron-ethyl, primisulfuron-methyl, nicosulfuron, amidosulfuron, cinosulfuron, imazosulfuron, rimsulfuron, halosulfuron-methyl, prosulfuron, ethametsulfuron-methyl, triflusulfuron-methyl, flazasulfuron, cyclosulfamuron, flupyrsulfuron, sulfosulfuron, azimsulfuron, ethoxysulfuron, oxasulfuron, iodosulfuron-methyl-sodium, foramsulfuron, mesosulfuron-methyl, trifloxysulfuron, tritosulfuron, orthosulfamuron, flucetosulfuron, and propyrisulfuron.

(19) Imidazolinone Herbicidal Compounds

Imazamethabenz-methyl, imazamethapyr, imazamox, imazapyr, imazaquin, imazethapyr, and the like.

(20) Sulfonamide Herbicidal Compounds

Flumetsulam, metosulam, diclosulam, florasulam, cloransulam-methyl, penoxsulam, and pyroxsulam. (21) Pyrimidinyloxy Benzoic Acid Herbicidal Compound

Pyrithiobac-sodium, bispyribac-sodium, pyriminobac-methyl, pyribenzoxim, pyriftalid, and pyrimisulfan.

(22) Other Herbicidal Compounds

bentazon, bromacil, terbacil, chlorthiamid, isoxaben, dinoseb, amitrole, cinmethylin, tridiphane, dalapon, diflufenzopyr-sodium, dithiopyr, thiazopyr, flucarbazone-sodium, propoxycarbazone-sodium., mefenacet, flufenacet, fentrazamide, cafenstrole, indanofan, oxaziclomefone, benfuresate, ACN, pyridate, chloridazon, norflurazon, flurtamone, diflufenican, picclinafen, beflubutamid, clomazone, amicarbazone, pinoxaden, pyraclonil, pyroxasulfone, thiencarbazone-methyl, aminocyclopyrachlor, ipfencarbazone, and methiozolin.

Active Ingredients of Synergists

Piperonyl butoxide, sesamex, sulfoxide, N-(2-ethylhexyl)-8,9,10-trinorborn-5-ene-2,3-dicarboximide (MGK 264), N-Declyimidazole, WARF-antiresistant, TBPT, TPP, IBP, PSCP, methyl iodide ($CH_3I$), t-Phenylbutenone, diethylmaleate, DMC, FDMC, ETP, and ETN.

Examples of pests for which the compound of the present invention has an effect include pathogenic endoparasites which are encountered with domestic animals, breeding stock, zoo animals, laboratory animals, animals used in a test, and pets. The compound of the present invention can be effective for tolerant species or general susceptible species of the endoparasites and for endoparasites in all developmental stages. It is possible to economically and simply rear animals by controlling the pathogenic endoparasites using the compound of the present invention, and it is possible to improve the yield rate of products (for example, meet, milk, wool, hides, eggs, and honey) derived from animals by reducing the disease rate and mortality in the animals. Pathogenic endoparasites include merozoas, trematodes, nematodes, and acanthocephalans. Particular examples of the endoparasites for which the compound of the present invention has an effect include the following endoparasites:

Pseudophyllidea, for example, Diphyllobothrium subgenus (Diphyllobothrium) (Diphyllobothrium spp.), Spirometra subgenus (Spirometra spp.), Schistocephalus subgenus (Schistosoma genus) (Schistocephalus spp.), Ligula subgenus (Ligula spp.), Bothridium subgenus (Bothridium spp.), Diplogonoporus subgenus (Diplogonoporus grandis) (Diphlogonoporus spp.);

Cyclophillidea, for example, Mesocestoides subgenus (Mesocestoides spp.), Anoplocephala subgenus (foliate tapeworm) (Anoplocephala spp.), Paranoplocephala subgenus (Paranoplocephala spp.), Moniezia subgenus (sheep tapeworm) (Moniezia spp.), Thysanosomsa subgenus (Thysanosomsa spp.), Thysaniezia subgenus (Thysaniezia spp.), Avitellina subgenus (Avitellina spp.), Stilesia subgenus (Stilesia spp.), Cittotaenia subgenus (Cittotaenia spp.), Andyra subgenus (Andyra spp.), Bertiella subgenus (Bertiella spp.), Taenia subgenus (Taenia spp.), Echinococcus subgenus (Echinococcus spp.), Hydatigera subgenus (Hydatigera spp.), Davainea subgenus (Davainea spp.), Raillietina subgenus (Raillietina spp.), hymenolepidid subgenus (hymenolepidid) (Hymenolepsis spp.); Echinolepis subgenus (Echinolepis spp.), Echinocotyle subgenus (Echinocotyle spp.), Diorchis subgenus (Diorchis spp.), Dipylidium subgenus (Dipylidium caninum) (Dipylidium spp.), Joyeuxiella subgenus (Joyeuxiella spp.), and Diplopylidium subgenus (Diplopylidium spp.);

Monogenea subclass, for example, Gyrodactylus subgenus (Gyrodactylus) (Gyrodactylus spp.), Dactylogyrus subgenus (Dactylogyrus spp.), and Polystoma subgenus (Polystoma spp.);

Digenea subclass, for example, Diplostomum subgenus (Diplostomum spp.), Posthodiplostomum subgenus (Posthodiplostomum spp.), Schistosoma subgenus (Schistosoma genus) (Schistosoma spp.), Trichobilharzia subgenus (Trichobilharzia spp.), Ornithobilharzia subgenus (Ornithobilharzia spp.), Austrobilharizia subgenus (Austrobilharizia spp.), Gigantobilharzia subgenus (Gigantobilharzia spp.), Leucochloridium subgenus (Leucochloridium spp.), Brachylaima subgenus (Brachylaima spp.), Echinostoma subgenus (Echinostoma genus) (Echinostoma spp.), Echinoparyphium subgenus (Echinoparyphium spp.), Echinochasmus subgenus (Echinochasmus spp.), Hypoderaeum subgenus (Hypoderaeum spp.), Fasciola subgenus (sheep liver fluke) (Fasciola spp.), Fasciolides subgenus (Fasciolides spp.), Fasciolopsis subgenus (giant intestinal fluke) (Fasciolcpsis spp.), Cyclocoelum subgenus (Cyclocoelum spp.), Typhlocoelum subgenus (Typhlocoelum spp.), Paramphistomum subgenus (Paramphistomum genus) (Paramphistomum spp.), Calicophoron subgenus (Calicophoron spp.), Cotylophoron subgenus (Cotylophoron spp.), Gigantocotyle subgenus (Gigantocotyle spp.), Fischoederius subgenus (Fischoederius spp.), Gastrothylacus subgenus (Gastrothylacus spp.), Notocotylus subgenus (Notocotylus spp.), Catatropis subgenus (Catatropis spp.), Plagiorchis subgenus (Plagiorchis spp.), Prosthogonimus subgenus (Prosthogonimus spp.), Dicrocoelium subgenus

(Dicrocoelium) (Dicrocoelium spp.), Eurytrema subgenus (pancreatic fluke). (Eurytrema spp.), Troglotrema subgenus (Troglotrema spp.), Paragonimus subgenus (Paragonimus genus) (Paragonimus spp.), Collyriclum subgenus (Collyriclum genus) (Collyriclum spp.), Nanophyetus subgenus (Nanophyetus spp.), Opisthorchis subgenus (Opisthorchis spp.), Clonorchis subgenus (Clonorchis sinensis) (Clonorchis spp.), Metorchis subgenus (Metorchis spp.), Heterophyes subgenus (Heterophyes genus) (Heterophyes spp.), and subclass of Metagonimus subgenus (Metagonimus spp.).

The compound of the present invention particularly controls the following endoparasites:

Enoplida, for example, Trichuris subgenus (whipworms) (Trichuris spp.), Capillaria subgenus (thread worms) (Capillaria spp.), Torikomosoidesu subgenus (Trichomosoides spp.), and Trichinella subgenus (Trichinella genus) (Trichinella spp.);
Rhabditia, for example, Micronema subgenus (Micronema spp.) and Strongyloides subgenus (Strongyloides spp.);
Strongylida, for example, Stronylus subgenus (strongyles) (Stronylus spp.), Triodontophorus subgenus (Triodontophorus spp.), Oesophagodontus subgenus (Oesophagodontus spp.), Trichonema subgenus (Trichonema spp.), Gyalocephalus subgenus (Gyalocephalus spp.), Cylindropharynx subgenus (Cylindropharynx spp.), Poteriostomum subgenus (Poteriostomum spp.), Cyclococercus subgenus (Cyclococercus spp.), Cylicostephanus subgenus (Cylicostephanus spp.), Oesophagostomum subgenus (Oesophagostomum genus) (Oesophagostomum spp.), Chabertia subgenus (Chabertia spp.), Stephanurus subgenus (Stephanurus dentatus) (Stephanurus spp.), Ancylostoma subgenus (old world hookworms) (Ancylostoma spp.), Uncinaria subgenus (Uncinaria spp.), and Bunostomum subgenus (Bunostomum spp.);
Globocephalus subgenus (Globocephalus spp.), Syngamus subgenus (syngamus worms) (Syngamus spp.), Cyathostoma subgenus (Cyathostoma spp.), Metastrongylus subgenus (lungworms) (Metastrongylus spp.), Dictyocaulus subgenus (Dictyocaulus spp.), Muellerius subgenus (Muellerius spp.), Protostrongylus subgenus (Protostrongylus spp.), Neostrongylus subgenus (Neostrongylus spp.), Cystocaulus subgenus (Cystocaulus spp.), Pneumostrongylus subgenus (Pneumostrongylus spp.), Spicocaulus subgenus (Spicocaulus spp.), Elaphostrongylus subgenus (Elaphostrongylus spp.), Parelaphostrongylus subgenus (Parelaphostrongylus spp.), Crenosoma subgenus (Crenosoma spp.), Paracrenosoma subgenus (Parelaphostrongylus spp.), Angiostrongylus subgenus (angiostrongylus worms) (Angiostrongylus spp.), Aelurosutrongylus subgenus (Aelurosutrongylus spp.), Filaroides subgenus (Filaroides spp.), Parafilaroides subgenus (Parafilaroides spp.), Trichostrongylus subgenus (hairworms) (Trichostrongylus spp.), Haemonchus subgenus (haemonchus worm) (Haemonchus spp.), Ostertagia subgenus (Ostertagia spp.), Marshallagia subgenus (Marshallagia spp.), Cooperia subgenus (Cooperia spp.), Nematodirus subgenus (nematode) (Nematodirus spp.), Hyostrongylus subgenus (Hyostrongylus spp.), Obeliscoides subgenus (Obeliscoides spp.), Amidostomum subgenus (Amidostomum spp.), and Ollulanus subgenus (Ollulanus spp.);
Oxyurida, for example, Oxyuris subgenus (horse pinworms) (Oxyuris spp.), Enterobius subgenus (pinworms) (Enterobius spp.), Passalurus subgenus (Passalurus spp.), Syphacia subgenus (Syphacia spp.), Aspiculuris subgenus (Aspiculuris spp.), and Heterakis subgenus (Heterakis spp.);
Ascaridia, for example, Ascaris subgenus (roundworms) (Ascaris spp.), Toxascaris subgenus (Toxascaris spp.), Toxocara subgenus (dog ascariasis) (Toxocara spp.), Parascaris subgenus (parascaris equorum) (Parascaris spp.), Anisakis Subgenus (Anisakis spp.), and Ascaridia subgenus (roundworms) (Ascaridia spp.);
Spirurida (spiruroids), for example, Gnathostoma subgenus (gnathostoma spinigerm) (Gnathostoma spp.), Physaloptera subgenus (Physaloptera spp.), Thelazia subgenus (Thelazia spp.), Gongylonema subgenus (Gongylonema spp.), Habronema subgenus (Habronema spp.), Parabronema subgenus (Parabronema spp.), Draschia subgenus (Draschia spp.), and Dracunculus subgenus (Guinea worms) (Dracunculus spp.);
Filariida, for example, Stephanofilaria subgenus (Stephanofilaria spp.), Parafilaria subgenus (Parafilaria spp.), Setaria Subgenus (Setaria spp.), Loa subgenus (Loa spp.), Dirofilaria subgenus (dog heartworms) (Dirofilaria spp.), Litomosoides subgenus (Litomosoides spp.), Brugia subgenus (Brugia spp.), Wuchereria subgenus (heartworms) (Wuchereria spp.), and Onchocerca Subgenus (Onchocerca spp.); and
Gigentorhynchida, for example, Filicollis subgenus (Filicollis spp.), Moniliforumis subgenus (Moniliforumis spp.), Macracanthorhynchus subgenus (Macracanthorhynchus spp.), and Prosthenorchis subgenus (Prosthenorchis spp.).

It is possible to cover the whole spectrum of the above-listed endoparasites when using the compounds of the present invention in combination as appropriate.

Particularly, examples of the endoparasites for which the compound of the present invention is highly effective include Toxocara cati (dog ascariasis), Toxascaris leonina, Ancylostoma tubaeforme (old world hookworms), Dipylidium caninum (Dipylidium caninum), Taenia taeniaeformis (hooked tapeworm), and Echinococcus multilocuraris.

Examples of the domestic animals and the breeding stock include, mammals, such as cattle, horses, sheep, pigs, goats, camels, water buffalo, donkeys, rabbits, fallow deer, and reindeer; animals, such as mink, chinchillas, or

raccoons, having fur; and birds, such as chicken, goose, turkey, duck, and ostrich.

Examples of the laboratory animals or the animals used in a test include mice, rats, guinea pigs, golden hamsters, dogs, and cats.

The pets include dogs and cats.

The administration of the compound of the present invention can be performed for both preventative and therapeutic purposes.

The compound of the present invention can be administered directly or in a form of a suitable formulation. Examples of the administration form include enteral administration, parenteral administration, topical administration, nasal administration, administration by treating an environment in which animals exist, or administration using shaped articles (for example, strips tablets, tape, collars, ear tags, bands for limbs, and marking devices) containing the compound of the present invention.

The enteral administration of the compound of the present invention is orally performed in forms of powder, suppositories, tablets, capsules, pastes, drinks, granules, liquid medicines, boluses, and feeds or drinking water to which medicinal substances are added. Specifically, the compound is applied to the skin through immersing, spraying, bathing, washing, pouring-on, spotting-on, or spraying. Alternately, the compound is parenterally administered by (intramuscular, subcutaneous, intravenous, or intraperitoneal) injection or in a form of being implanted.

Examples of the appropriate formula include the following.

Liquid medicines for injection, oral liquid medicines, concentrates for oral administration after dilution, liquid medicines for use on skin or in body cavities, pouring-on or spotting-on formulations, and gels; emulsions and suspensions for oral or topical administration, and for injection; semi-solid formulations; formulations in which an active substance is incorporated in an ointment base or in oil-in-water or water-in-oil emulsion base; solid formulations, such as powder, premix or concentrates, granules, pellets, tablets, boluses, and capsules; and aerosols, inhalants, and shaped articles containing an active substance.

The liquid medicines for injection are intravenously, intramuscularly, or subcutaneously administered.

The oral liquid medicines are used directly. The concentrates are orally used after being diluted in advance to its concentration to be used.

The liquid medicines used in the skin are administered by dripping, coating, fractioning, spraying, spraying, immersing, bathing, or washing.

The gels are administered to or coated on the skin, or introduced into body cavities.

The pouring-on or spotting-on formulations are poured or sprayed on a limited area of the skin so that the compound of the present invention transmits through the skin and systemically acts, or is spread on a surface of the body.

The emulsions can be orally used or used in the skin in a form of injection. Either of an oil-in-water type and a water-in-oil type is used as the emulsion.

The suspensions can be orally used or used in the skin in the form of injection.

The semi-solid formulations can be administered orally or on the skin.

The solid formulations can be prepared by mixing the compound of the present invention with a suitable carrier by adding an auxiliary agent to the mixture as necessary to be formed in a desired shape. The formulations of the present invention can further contain an active substance as necessary.

## Examples

Hereinafter, the present invention will be described in more detail with reference to examples, but is not limited to these examples.

(Production Example 1)

Production of 2,6-dichloro-N-((4-phenyl-1-(2-(trifluoromethyl)phenethyl)piperidin-4-yl)methyl)isonicotinamide

(1) Production of 4-phenyl-1-(2-(trifluoromethyl)phenethyl)piperidine-4-carbonitrile

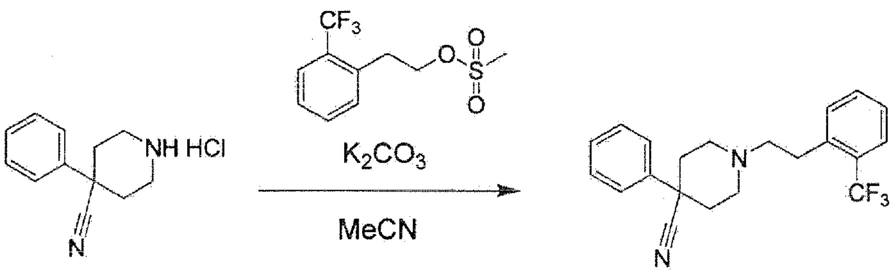


4160 mg of 2-(trifluoromethyl)phenethyl methanesulfonate and 4930 mg of potassium carbonate were added to a mixture of 200 ml of acetonitrile and 3452 mg of 4-cyano-4-phenylpiperidine hydrochloride at room temperature, and the mixture was stirred for 5 hours while being heated and refluxed. After filtration of the reaction mixture, the filtrate was concentrated under reduced pressure. The residue was subjected to silicagel chromatography to obtain 4630 mg of 4-phenyl-1-(2-(trifluoromethyl)phenethyl)piperidine-4-carbonitrile. $^1$H-NMR ($CDCl_3$) $\delta$: 7.66-7.62 (1H, m), 7.55-7.46 (3H, m), 7.44-7.29 (5H, m), 3.15-3.08 (2H, m), 3.05-2.99 (2H, m), 2.76-2.69 (2H, m), 2.67-2.59 (2H, m), 2.18-2.11 (4H, m).

(2) Production of 4-aminomethyl-4-phenyl-1-(2-(trifluoromethyl)phenethyl)piperidine

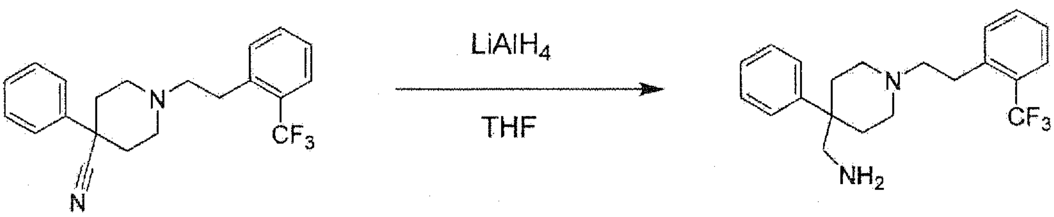


1225 mg of lithium aluminum hydride was added to a mixture of 200 ml of tetrahydrofuran and 4630 mg of 4-phenyl-1-(2-(trifluoromethyl)phenethyl)piperidine-4-carbonitrile under ice-cooling, and the mixture was stirred for 10 hours at room temperature. Then, 50 ml of water and 50 ml of 10% sodium hydroxide solution were added thereto and the reaction mixture was filtered. Thereafter, a liquid separation operation was performed using tert-butyl methyl ether and an organic layer was concentrated to obtain 3620 mg of 4-aminomethyl-4-phenyl-1-(2-(trifluoromethyl)phenethyl)piperidine.
$^1$H-NMR ($CDCl_3$) $\delta$: 7.61-7.57 (1H, m), 7.46-7.41 (1H, m), 7.40-7.20 (7H, m), 2.99-2.92 (2H, m), 2.81-2.70 (4H, m), 2.56-2.49 (2H, m), 2.35-2.22 (4H, m), 1.91-1.82 (2H, m), 0.70 (2H, br s).

(3) Production of 2,6-dichloro-N-((4-phenyl-1-(2-(trifluoromethyl)phenethyl)piperidin-4-yl)methyl)isonicotinamide (Hereinafter, Also Referred to as compound 1 of the present invention)

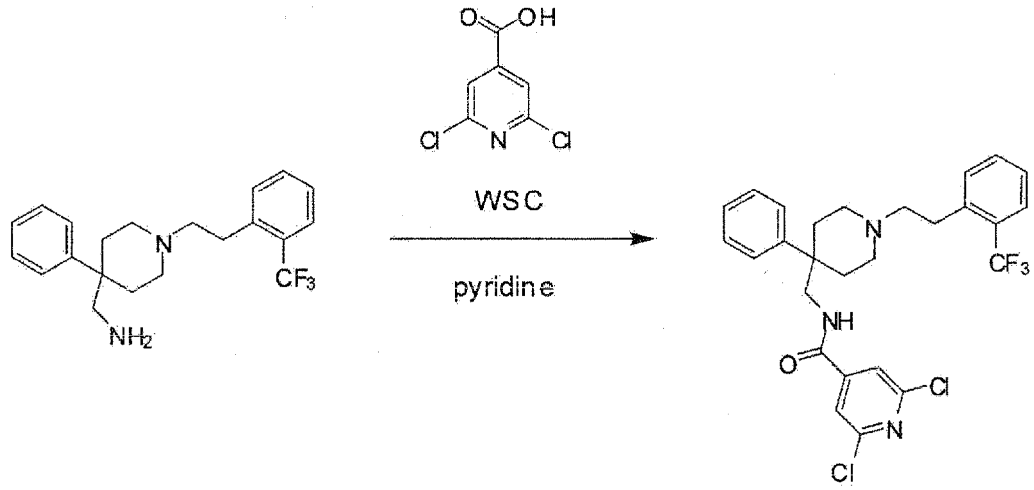


281 mg of 2,6-dichloroisonicotinic acid and 364 mg of 1-ethyl-3-(3-dimethylaminopropyl)carbodiimide hydrochloride were added to a mixture of 30 ml of pyridine and 530 mg of 4-aminomethyl-4-phenyl-1-(2-(trifluoromethyl)phenethyl)piperidine at room temperature, and the mixture was stirred for 5 hours at 90°C. 30 ml of water was added to the reaction mixture and a liquid separation operation was performed using ethyl acetate, the n the organic layer was concentrated. The residue was subjected to silicagel chromatography to obtain a compound 1 of the present invention (300 mg).
$^1$H-NMR ($CDCl_3$) $\delta$: 7.62-7.58 (1H, m), 7.48-7.42 (3H, m), 7.40-7.23 (7H, m), 5.65-5.57 (1H, m), 3.68-3.58 (2H, m), 3.00-2.91 (2H, m), 2.80-2.69 (2H, m), 2.62-2.52 (2H, m), 2.50-2.40 (2H, m), 2.29-2.18 (2H, m), 2.05-1.95 (2H, m).

(Production Example 2) Production of 2-chloro-N-((1-phenethyl-4-phenylpiperidin-4-yl)methyl)isonicotinamide

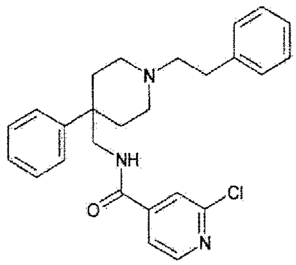

The compound of the subtitle (hereinafter, written as a compound 2 of the present invention) was obtained by performing the same reaction as that in Production Example 1 using phenethyl methanesulfonate instead of 2-(trifluoromethyl)phenethyl methanesulfonate and using 2-chloroisonicotinic acid instead of 2,6-dichloroisonicotinic acid.
MS-:432

(Production Example 3) Production of 2-chloro-N-((4-phenyl-1-(2-(trifluoromethyl)phenethyl)piperidin-4-yl)methyl)isonicotinamide

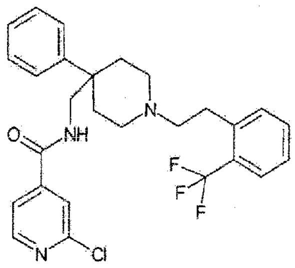

The compound of the subtitle (hereinafter, written as a compound 3 of the present invention) was obtained by performing the same reaction as that in Production Example 1 using 2-chloroisonicotinic acid instead of 2,6-dichloroisonicotinic acid.
$^1$H-NMR ($CDCl_3$) $\delta$: 8.45-8.43 (1H, m), 7.62-7.58 (1H, m), 7.48-7.42 (4H, m), 7.41-7.37 (2H, m), 7.35-7.27 (4H, m), 5.63 (1H, s), 3.68-3.63 (2H, m), 2.99-2.92 (2H, m), 2.79-2.70 (2H, m), 2.60-2.53 (2H, m), 2.52-2.42 (2H, m), 2.28-2.20 (2H, m), 2.06-1.96 (2H, m).

(Production Example 4) Production of 2-chloro-N-((4-phenyl-1-(3-(trifluoromethyl)phenethyl)piperidin-4-yl)methyl)isonicotinamide

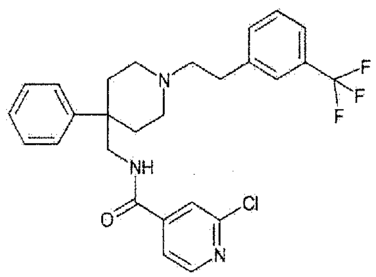

The compound of the subtitle (hereinafter, written as a compound 4 of the present invention) was obtained by performing the same reaction as that in Production Example 1 using 3-(trifluoromethyl)phenethyl methanesulfonate instead of 2-(trifluoromethyl)phenethyl methanesulfonate and using 2-chloroisonicotinic acid instead of 2,6-dichloroisonicotinic acid.
$^1$H-NMR ($CDCl_3$) $\delta$: 8.46-8.43 (1H, m), 7.51-7.41 (5H, m), 7.41-7.36 (4H, m), 7.35-7.29 (2H, m), 5.83-5.74 (1H, m), 3.69-3.63 (2H, m), 2.92-2.77 (4H, m), 2.68-2.59 (2H, m), 2.56-2.44 (2H, m), 2.32-2.20 (2H, m), 2.15-2.03 (2H, m).

(Production Example 5)

Production of N-((1-phenethyl-4-phenylpiperidin-4-yl)methyl)benzamide

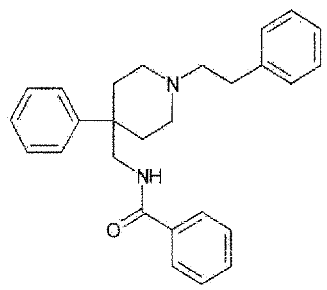

The compound of the subtitle (hereinafter, written as a compound 5 of the present invention) was obtained by performing the same reaction as that in Production Example 1 using phenethyl methanesulfonate instead of 2-(trifluoromethyl)phenethyl methanesulfonate and using benzoic acid instead of 2,6-dichloroisonicotinic acid.
$^1$H-NMR ($CDCl_3$) δ: 7.59-7.54 (2H, m), 7.48-7.34 (7H, m), 7.33-7.24 (3H, m), 7.21-7.15 (3H, m), 5.73-5.63 (1H, m), 3.73-3.65 (2H, m), 2.84-2.71 (4H, m), 2.64-2.56 (2H, m), 2.55-2.46 (2H, m), 2.28-2.17 (2H, m), 2.10-2.01 (2H, m).

(Production Example 6) Production of 4-chloro-N-((1-phenethyl-4-phenylpiperidin-4-yl)methyl)benzamide

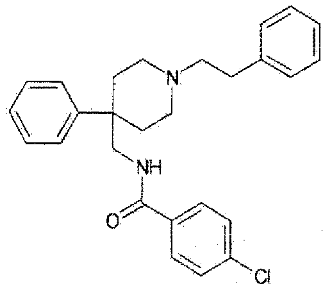

The compound of the subtitle (hereinafter, written as a compound 6 of the present invention) was obtained by performing the same reaction as that in Production Example 1 using phenethyl methanesulfonate instead of 2-(trifluoromethyl)phenethyl methanesulfonate and using 4-chlorobenzoic acid instead of 2,6-dichloroisonicotinic acid.
$^1$H-NMR ($CDCl_3$) δ: 7.51-7.47 (2H, m), 7.45-7.24 (10H, m), 7.20-7.16 (2H, m), 5.65-5.56 (1H, m), 3.71-3.63 (2H, m), 2.86-2.70 (4H, m), 2.63-2.54 (2H, m), 2.54-2.43 (2H, m), 2.27-2.17 (2H, m), 2.09-1.99 (2H, m).

(Production Example 7) Production of N-((1-phenethyl-4-phenylpiperidin-4-yl)methyl)picolinamide

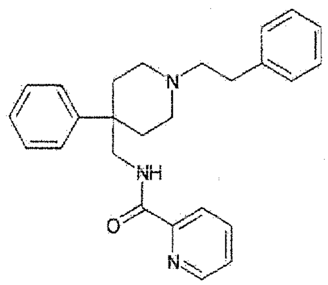

The compound of the subtitle (hereinafter, written as a compound 7 of the present invention) was obtained by performing the same reaction as that in Production Example 1 using phenethyl methanesulfonate instead of 2-(trifluoromethyl)phenethyl methanesulfonate and using picolinic acid instead of 2,6-dichloroisonicotinic acid.
$^1$H-NMR ($CDCl_3$) δ: 8.47-8.44 (1H, m), 8.17-8.13 (1H, m), 7.88-7.79 (2H, m), 7.42-7.38 (4H, m), 7.31-7.23 (3H, m), 7.20-7.14 (3H, m), 3.69-3.63 (2H, m), 2.82-2.74 (4H, m), 2.61-2.54 (2H, m), 2.49-2.39 (2H, m), 2.29-2.21 (2H, m), 2.09-2.01 (2H, m).

(Production Example 8) Production of N-((1-phenethyl-4-phenylpiperidin-4-yl)methyl)nicotinamide

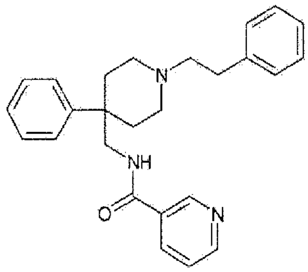

The compound of the subtitle (hereinafter, written as a compound 8 of the present invention) was obtained by performing the same reaction as that in Production Example 1 using phenethyl methanesulfonate instead of 2-(trifluoromethyl)phenethyl methanesulfonate and using nicotinic acid instead of 2,6-dichloroisonicotinic acid.
$^1$H-NMR ($CDCl_3$) δ: 8.71-8.67 (2H, m), 7.98-7.94 (1H, m), 7.47-7.37 (5H, m), 7.36-7.23 (3H, m), 7.21-7.15 (3H, m), 5.69-5.62 (1H, m), 3.72-3.67 (2H, m), 2.83-2.70 (4H, m), 2.63-2.55 (2H, m), 2.53-2.44 (2H, m), 2.28-2.19 (2H, m), 2.09-2.00 (2H, m).

(Production Example 9) Production of N-((1-phenethyl-4-phenylpiperidin-4-yl)methyl)isonicotinamide

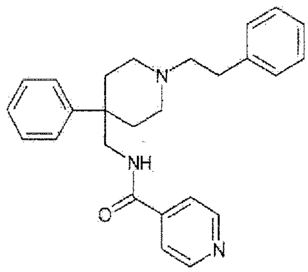

The compound of the subtitle (hereinafter, written as a compound 9 of the present invention) was obtained by performing the same reaction as that in Production Example 1 using phenethyl methanesulfonate instead of 2-(trifluoromethyl)phenethyl methanesulfonate and using isonicotinic acid instead of 2,6-dichlbroisonicotinic acid.
$^1$H-NMR ($CDCl_3$) δ: 8.69-8.66 (2H, m), 7.48-7.42 (2H, m), 7.42-7.36 (4H, m), 7.35-7.24 (4H, m), 7.21-7.15 (2H, m), 5.83-5.75 (1H, m), 3.70-3.66 (2H, m), 2.85-2.75 (4H, m), 2.65-2.58 (2H, m), 2.56-2.47 (2H, m), 2.29-2.20 (2H, m), 2.12-2.04 (2H, m).

(Production Example 10) Production of 2-chloro-N-((4-phenyl-1-(4-(trifluoromethyl)phenethyl)piperidin-4-yl)methyl)isonicotinamide

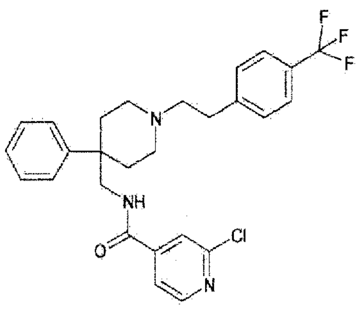

The compound of the subtitle (hereinafter, written as a compound 10 of the present invention) was obtained by performing the same reaction as that in Production Example 1 using 4-(trifluoromethyl)phenethyl methanesulfonate instead of 2-(trifluoromethyl)phenethyl methanesulfonate and using 2-chloroisonicotinic acid instead of 2,6-dichloroisonicotinic acid.

MS+:502, MS-:500.

(Production Example 11) Production of N-((4-phenyl-1-(2-(trifluoromethyl)phenethyl)piperidin-4-yl)methyl)benzamide

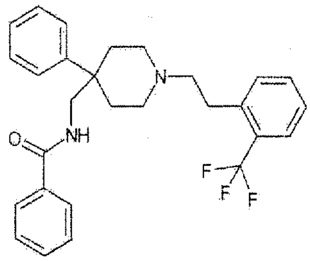

The compound of the subtitle (hereinafter, written as a compound 11 of the present invention) was obtained by performing the same reaction as that in Production Example 1 using benzoic acid instead of 2,6-dichloroisonicotinic acid.
$^1$H-NMR ($CDCl_3$) δ: 7.62-7.58 (1H, m), 7.58-7.53 (2H, m), 7.49-7.26 (11H, m), 5.70-5.61 (1H, m), 3.73-3.64 (2H, m), 3.00-2.92 (2H, m), 2.79-2.71 (2H, m), 2.62-2.54 (2H, m), 2.53-2.44 (2H, m), 2.27-2.18 (2H, m), 2.09-1.99 (2H, m).

(Production Example 12) Production of 2-chloro-N-((4-phenyl-1-(2-(trifluoromethyl)phenethyl)piperidin-4-yl)methyl)benzamide

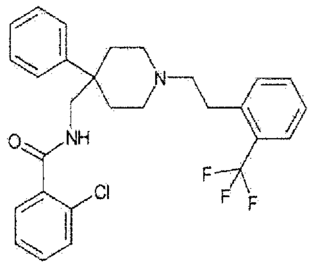

The compound of the subtitle (hereinafter, written as a compound 12 of the present invention) was obtained by performing the same reaction as that in Production Example 1 using 2-chlorobenzoic acid instead of 2,6-dichloroisonicotinic acid.
$^1$H-NMR ($CDCl_3$) δ: 7.62-7.58 (1H, m), 7.58-7.54 (1H, m), 7.48-7.40 (2H, m), 7.40-7.37 (3H, m), 7.36-7.33 (2H, m), 7.32-7.29 (2H, m), 7.2.9-7.24 (2H, m), 5.83-5.76 (1H, m), 3.76-3.70 (2H, m), 3.01-2.92 (2H, m), 2.81-2.72 (2H, m), 2.62-2.55 (2H, m), 2.55-2.47 (2H, m), 2.29-2.21 (2H, m), 2.0.9-72.02 (2H, m).

(Production Example 13) Production of 3-chloro-N-((4-phenyl-1-(2-(trifluoromethyl)phenethyl)piperidin-4-yl)methyl)benzamide

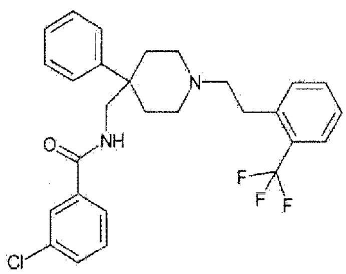

The compound of the subtitle (hereinafter, written as a compound 13 of the present invention) was obtained by performing the same reaction as that in Production Example 1 using 3-chlorobenzoic acid instead of 2,6-dichloroisonicotinic acid.
$^1$H-NMR ($CDCl_3$) δ: 7.61-7.55 (2H, m), 7.47-7.36 (7H, m), 7.35-7.25 (4H, m), 5.65-5.56 (1H, m), 3.70-3.61 (2H, m), 3.00-2.91 (2H, m), 2.79-2.70 (2H, m), 2.62-2.52 (2H, m), 2.51-2.42 (2H, m), 2.28-2.17 (2H, m), 2.07-1.98 (2H, m).

(Production Example 14)

Production of 4-chloro-N-((4-phenyl-1-(2-(trifluoromethyl)phenethyl)piperidin-4-yl)methyl)benzamide

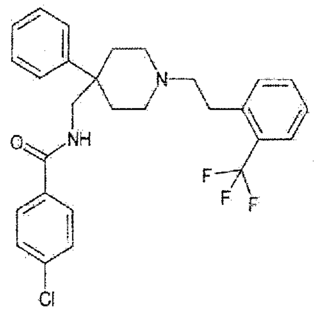

The compound of the subtitle (hereinafter, written as a compound 14 of the present invention) was obtained by performing the same reaction as that in Production Example 1 using 4-chlorobenzoic acid instead of 2,6-dichloroisonicotinic acid.
$^1$H-NMR ($CDCl_3$) δ: 7.61-7.58 (1H, m), 7.51-7.47 (2H, m), 7.47-7.38 (5H, m), 7.36-7.32 (3H, m), 7.31-7.27 (2H, m), 5.65-5.60 (1H, m), 3.68-3.63 (2H, m), 3.01-2.93 (2H, m), 2.82-2.74 (2H, m), 2.63-2.55 (2H, m), 2.55-2.45 (2H, m), 2.28-2.18 (2H, m), 2.10-2.01 (2H, m).

(Production Example 15) Production of 2-fluoro-N-((4-phenyl-1-(2-(trifluoromethyl)phenethyl)piperidin-4-yl)methyl)isonicotinamide

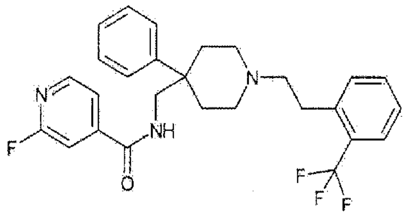

The compound of the subtitle (hereinafter, written as a compound 15 of the present invention) was obtained by performing the same reaction as that in Production Example 1 using 2-fluoro-isonicotinic acid instead of 2,6-dichloroisonicotinic acid.
$^1$H-NMR ($CDCl_3$) δ: 8.29-8.25 (1H, m), 7.62-7.57 (1H, m), 7.48-7.42 (3H, m), 7.41-7.36 (2H, m), 7.35-7.27 (3H, m), 7.24-7.21 (1H, m), 7.08-7.04 (1H, m), 5.71-5.63 (1H, m), 3.69-3.63 (2H, m), 3.00-2.91 (2H, m), 2.81-2.70 (2H, m), 2.61-2.54 (2H, m), 2.52-2.42 (2H, m), 2.29-2.18 (2H, m), 2.08-1.96 (2H, m).

(Production Example 16) Production of N-((4-phenyl-1-(2-(trifluoromethyl)phenethyl)piperidin-4-yl)methyl)picolinamide

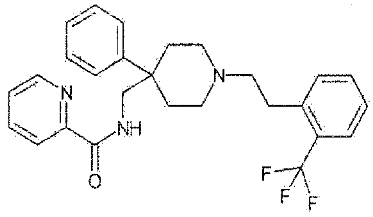

The compound of the subtitle (hereinafter, written as a compound 16 of the present invention) was obtained by performing the same reaction as that in Production Example 1 using picolinic acid instead of 2,6-dichloroisonicotinic acid.
$^1$H-NMR ($CDCl_3$) δ: 8.47-8.44 (1H, m), 8.17-8.13 (1H, m), 7.88-7.79 (2H, m), 7.61-7.57 (1H, m), 7.46-7.36 (6H, m), 7.34-7.27 (2H, m), 3.67-3.63 (2H, m), 2.98-2.92 (2H, m), 2.81-2.73 (2H, m), 2.58-2.52 (2H, m), 2.46-2.38 (2H, m), 2.30-2.21 (2H, m), 2.08-2.00 (2H, m).

(Production Example 17) Production of 3,5-dichloro-N-((4-phenyl-1-(2-(trifluoromethyl)phenethyl)piperidin-4-yl)methyl)benzamide

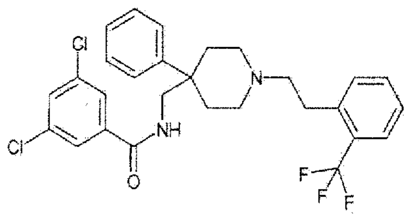

The compound of the subtitle (hereinafter, written as a compound 17 of the present invention) was obtained by performing the same reaction as that in Production Example 1 using 3,5-dichlorobenzoic acid instead of 2,6-dichloroisonicotinic acid.
$^1$H-NMR ($CDCl_3$) δ: 7.61-7.58 (1H, m), 7.48-7.42 (4H, m), 7.41-7.38 (4H, m), 7.35-7.27 (3H, m), 5.59-5.52 (1H, m), 3.66-3.60 (2H, m), 2.99-2.92 (2H, m), 2.79-2.70 (2H, m), 2.61-2.53 (2H, m), 2.52-2.42 (2H, m), 2.28-2.19 (2H, m), 2.05-1.97 (2H, m).

(Production Example 18) Production of N-((4-phenyl-1-(2-(trifluoromethyl)phenethyl)piperidin-4-yl)methyl)nicotinamide

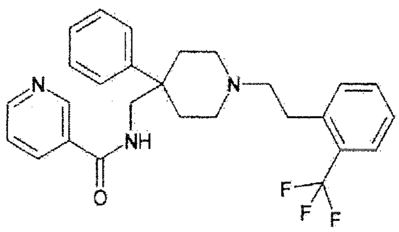

The compound of the subtitle (hereinafter, written as a compound 18 of the present invention) was obtained by performing the same reaction as that in Production Example 1 using nicotinic acid instead of 2,6-dichloroisonicotinic acid.
$^1$H-NMR ($CDCl_3$) δ: 8.72-8.67 (2H, m), 7.99-7.94 (1H, m), 7.62-7.58 (1H, m), 7.48-7.37 (5H, m), 7.37-7.27 (4H, m), 5.71-5.64 (1H, m), 3.72-3.67 (2H, m), 3.02-2.94 (2H, m), 2.82-2.74 (2H, m), 2.63-2.55 (2H, m), 2.55-2.46 (2H, m), 2.31-2.21 (2H, m), 2.10-2.01 (2H, m).

(Production Example 19) Production of N-((4-phenyl-1-(2-(trifluoromethyl)phenethyl)piperidin-4-yl)methyl)isonicotinamide

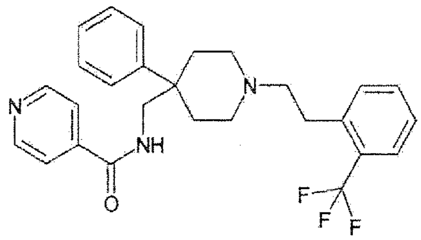

The compound of the subtitle (hereinafter, written as a compound 19 of the present invention) was obtained by performing the same reaction as that in Production Example 1 using isonicotinic acid instead of 2,6-dichloroisonicotinic acid.
$^1$H-NMR ($CDCl_3$) δ: 8.70-8.67 (2H, m), 7.62-7.58 (1H, m), 7.49-7.28 (10H, m), 5.83-5.69 (1H, m), 3.75-3.62 (2H, m), 3.09-2.93 (2H, m), 2.88-2.75 (2H, m), 2.68-2.46 (4H, m), 2.33-2.21 (2H, m), 2.15-2.01 (2H, m).

(Production Example 20) Production of 2,6-dichloro-N-((1-phenethyl-4-phenylpiperidin-4-yl)methyl)isonicotinamide

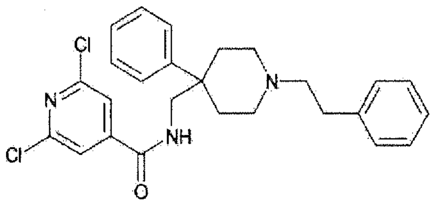

The compound of the subtitle (hereinafter, written as a compound 20 of the present invention) was obtained by performing the same reaction as that in Production Example 1 using phenethyl methanesulfonate instead of 2-(trifluoromethyl)phenethyl methanesulfonate.
MS-:466.

(Production Example 21) Production of 3-chloro-N-((4-phenyl-1-(2-(trifluoromethyl)phenethyl)piperidin-4-yl)methyl)picolinamide

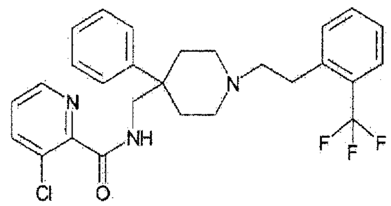

The compound of the subtitle (hereinafter, written as a compound 21 of the present invention) was obtained by performing the same reaction as that in Production Example 1 using 3-chloro-picolinic acid instead of 2,6-dichloroisonicotinic acid.
$^1$H-NMR ($CDCl_3$) δ: 8.38-8.35 (1H, m), 7.80-7.76 (1H, m), 7.61-7.57 (1H, m), 7.49-7.37 (7H, m), 7.35-7.27 (2H, m), 3.66-3.61 (2H, m), 2.98-2.90 (2H, m), 2.81-2.72 (2H, m), 2.59-2.52 (2H, m), 2.49-2.39 (2H, m), 2.29-2.20 (2H, m), 2.10-2.00 (2H, m).

(Production Example 22) Production of 2-chloro-N-((4-(4-chlorophenyl)-1-(2-(trifluoromethyl)phenethyl)piperidin-4-yl)methyl)benzamide

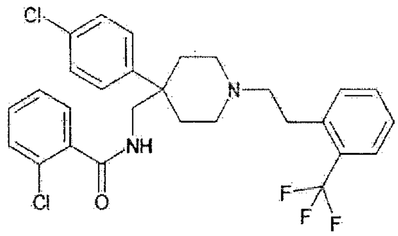

The compound of the subtitle (hereinafter, written as a compound 22 of the present invention) was obtained by performing the same reaction as that in Production Example 1 using 4-cyano-4-(4-chlorophenyl)piperidine hydrochloride instead of 4-cyano-4-phenylpiperidine hydrochloride and using 2-chlorobenzoic acid instead of 2,6-dichloroisonicotinic acid.
$^1$H-NMR ($CDCl_3$) δ: 7.62-7.55 (2H, m), 7.48-7.43 (1H, m), 7.40-7.27 (9H, m), 5.84-5.75 (1H, m), 3.76-3.65 (2H, m), 3.01-2.89 (2H, m), 2.80-2.69 (2H, m), 2.62-2.52 (2H, m), 2.52-2.43 (2H, m), 2.26-2.14 (2H, m), 2.10-1.99 (2H, m).

(Production Example 23)

Production of 2-chloro-N-((4-(3-chlorophenyl)-1-(2-(trifluoromethyl)phenethyl)piperidin-4-yl)methyl)benzamide

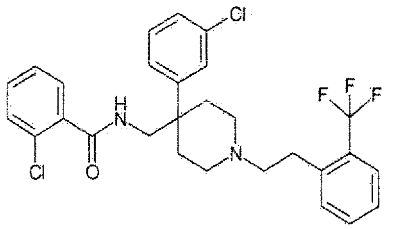

The compound of the subtitle (hereinafter, written as a compound 23 of the present invention) was obtained by performing the same reaction as that in Production Example 1 using 4-cyano-4-(3-chlorophenyl)piperidine hydrochloride instead of 4-cyano-4-phenylpiperidine hydrochloride and using 2-chlorobenzoic acid instead of 2,6-dichloroisonicotinic acid.
$^1$H-NMR ($CDCl_3$) δ: 7.62-7.57 (2H, m), 7.48-7.43 (1H, m), 7.40-7.22 (9H, m), 5.88-5.81 (1H, m), 3.76-3.69 (2H, m), 3.01-2.92 (2H, m), 2.81-2.72 (2H, m), 2.63-2.56 (2H, m), 2.54-2.44 (2H, m), 2.26-2.15 (2H, m), 2.10-2.00 (2H, m).

(Production Example 24) Production of 2-chloro-N-((4-phenyl-1-(2-(trifluoromethyl)phenethyl)piperidin-4-yl)methyl)nicotinamide

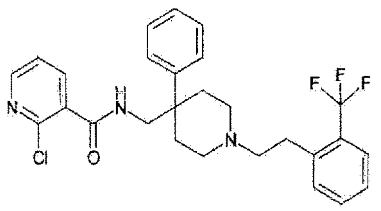

The compound of the subtitle (hereinafter, written a s a compound 24 of the present invention) was obtained by p erforming the same reaction as that in Production Example 1 using 2-chloro-nicotinic acid instead of 2,6-dichloroisonicotinic acid.
MS+:502

(Production Example 25) Production of 2-chloro-N-((4-(2-chlorophenyl)-1-(2-(trifluoromethyl)phenethyl)piperidin-4-yl)methyl)benzamide

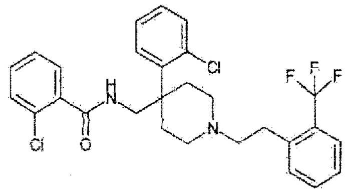

The compound of the subtitle (hereinafter, written as a compound 25 of the present invention) was obtained by performing the same reaction as that in Production Example 1 using 4-cyano-4-(2-chlorophenyl)piperidine hydrochloride instead of 4-cyano-4-phenylpiperidine hydrochloride and using 2-chlorobenzoic acid instead of 2,6-dichloroisonicotinic acid.
MS+:535

(Production Example 26) Production of 2,6-dichloro-N-((1-(2-methoxyphenethyl)-4-phenylpiperidin-4-yl)methyl)isonicotinamide

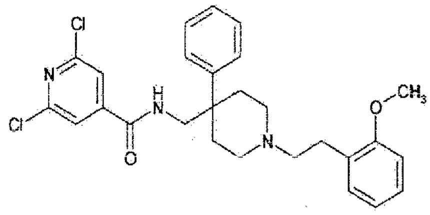

The compound of the subtitle (hereinafter, written as a compound 26 of the present invention) was obtained by performing the same reaction as that in Production Example 1 using 2-methoxyphenethyl methanesulfonate instead of 2-(trifluoromethyl)phenethyl methanesulfonate.
MS+:499

(Production Example 27) Production of 2,6-dichloro-N-((1-(2-methylphenethyl)-4-phenylpiperidin-4-yl)methyl)isonicotinamide

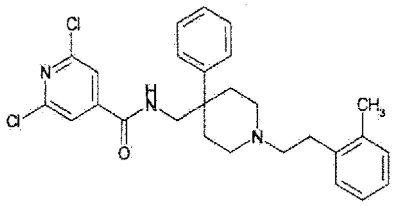

The compound of the subtitle (hereinafter, written a s a compound 27 of the present invention) was obtained by p erforming the same reaction as that in Production Example 1 using 2-methylphenethyl methanesulfonate instead of 2-(trifluoromethyl)phenethyl methanesulfonate.
MS+:483

(Production Example 28) Production of 2-methoxy-N-((4-phenyl-1-(2-(trifluoromethyl)phenethyl)piperidin-4-yl)methyl)benzamide

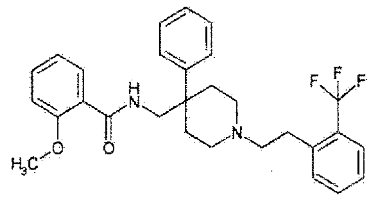

The compound of the subtitle (hereinafter, written as a compound 28 of the present invention) was obtained by performing the same reaction as that in Production Example 1 using 2-methoxybenzoic acid instead of 2,6-dichloroisonicotinic acid.
$^1$H-NMR ($CDCl_3$) δ: 8.21-8.17 (1H, m), 7.63-7.53 (2H, m), 7.47-7.25 (8H, m), 7.07-7.01 (1H, m), 6.88-6.81 (1H, m), 3.81-3.75 (2H, m), 3.58 (3H, s), 3.00-2.93 (2H, m), 2.77-2.68 (2H, m), 2.64-2.49 (4H, m), 2.24-2.15 (2H, m), 2.08-1.99 (2H, m).

(Production Example 29) Production of 2,6-dichloro-N-((1-(2-chlorophenethyl)-4-phenylpiperidin-4-yl)methyl)isonicotinamide

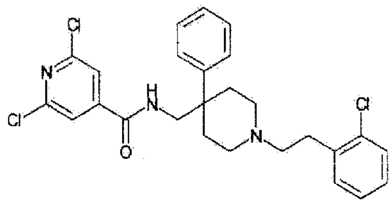

The compound of the subtitle (hereinafter, written as a compound 29 of the present invention) was obtained by performing the same reaction as that in Production Example 1 using 2-chlorophenethyl methanesulfonate instead of 2-(trifluoromethyl)phenethyl methanesulfonate.
$^1$H-NMR ($CDCl_3$) δ: 7.48-7.43 (2H, m), 7.41-7.36 (2H, m), 7.36-7.27 (4H, m), 7.23-7.11 (3H, m), 5.63-5.57 (1H, m), 3.68-3.61 (2H, m), 2.96-2.88 (2H, m), 2.80-2.71 (2H, m), 2.62-2.53 (2H, m), 2.52-2.42 (2H, m), 2.29-2.19 (2H, m), 2.07-1.95 (2H, m).

(Production Example 30)

Production of 2-chloro-N-((1-(2-methylphenethyl)-4-phenylpiperidin-4-yl)methyl)benzamide

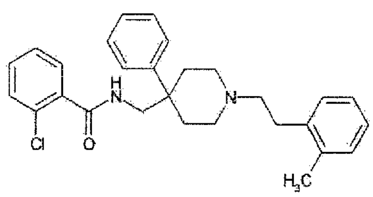

The compound of the subtitle (hereinafter, written as a compound 30 of the present invention) was obtained by performing the same reaction as that in Production Example 1 using 2-methylphenethyl methanesulfonate instead of 2-(trifluoromethyl)phenethyl methanesulfonate and using 2-chlorobenzoic acid instead of 2,6-dichloroisonicotinic acid.
MS+:448

(Production Example 31) Production of 2-chloro-N-((1-(2-chlorophenethyl)-4-phenylpiperidin-4-yl)methyl)benzamid

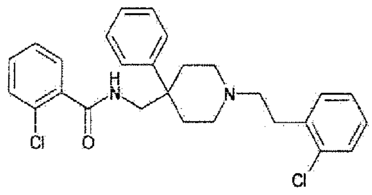

The compound of the subtitle (hereinafter, written as a compound 31 of the present invention) was obtained by performing the same reaction as that in Production Example 1 using 2-chlorophenethyl methanesulfonate instead of 2-(trifluoromethyl)phenethyl methanesulfonate and using 2-chlorobenzoic acid instead of 2,6-dichloroisohicotinic acid.
$^1$H-NMR ($CDCl_3$) δ: 7.58-7.54 (1H, m), 7.42-7.35 (5H, m), 7.33-7.21 (5H, m), 7.19-7.10 (2H, m), 5.83-5.77 (1H, m), 3.76-3.71 (2H, m), 2.96-2.90 (2H, m), 2.81-2.73 (2H, m), 2.61-2.48 (4H, m), 2.29-2.21 (2H, m), 2.09-2.02 (2H, m).

(Production Example 32) Production of N-((1-(2,3-dichlorophenethyl)-4-phenylpiperidin-4-yl)methyl)-2-methoxy-benzamide

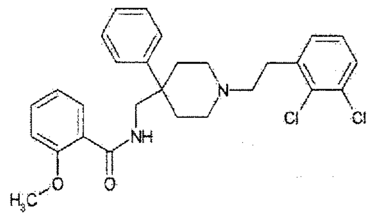

The compound of the subtitle (hereinafter, written as a compound 32 of the present invention) was obtained by performing the same reaction as that in Production Example 1 using 2,3-dichlorophenethyl methanesulfonate instead of 2-(trifluoromethyl)phenethyl methanesulfonate and using 2-methoxybenzoic acid instead of 2,6-dichloroisonicotinic acid.
$^1$H-NMR ($CDCl_3$) δ: 8.21-8.17 (1H, m), 7.59-7.53 (1H, m), 7.44-7.36 (5H, m), 7.33-7.27 (2H, m), 7.16-7.01 (3H, m), 6.87-6.82 (1H, m), 3.80-3.75 (2H, m), 3.57 (3H, s), 2.99-2.93 (2H, m), 2.78-2.69 (2H, m), 2.62-2.51 (4H, m), 2.22-2.14 (2H, m), 2.07-1.99 (2H, m).

(Production Example 33) Production of 2,6-dichloro-N-((1-(2,3-dichlorophenethyl)-4-phenylpiperidin-4-yl)methyl)isonicotinamide

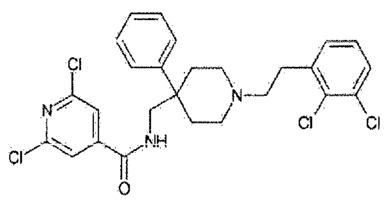

The compound of the subtitle (hereinafter, written as a compound 33 of the present invention) was obtained by performing the same reaction as that in Production Example 1 using 2,3-dichlorophenethyl methanesulfonate instead of 2-(trifluoromethyl)phenethyl methanesulfonate.
$^1$H-NMR ($CDCl_3$) δ: 7.48-7.42 (2H, m), 7.40-7.36 (2H, m), 7.35-7.29 (4H, m), 7.18-7.08 (2H, m), 5.72-5.66 (1H, m), 3.65-3.61 (2H, m), 2.98-2.91 (2H, m), 2.79-2.71 (2H, m), 2.59-2.53 (2H, m), 2.50-2.41 (2H, m), 2.19-2.11 (2H, m), 2.03-1.95 (2H, m).

(Production Example 34) Production of 2,6-dimethoxy-N-((4-phenyl-1-(2-(trifluoromethyl)phenethyl)piperidin-4-yl)methyl)benzamide

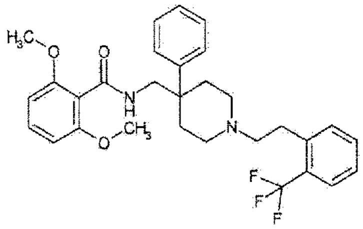

The compound of the subtitle (hereinafter, written as a compound 34 of the present invention) was obtained by performing the same reaction as that in Production Example 1 using 2,6-dimethoxybenzoic acid instead of 2,6-dichloroisonicotinic acid.
MS+:527.

(Production Example 35) Production of N-((4-phenyl-1-(2-(trifluoromethyl)phenethyl)piperidin-4-yl)methyl)-2-trifluoromethyl-benzamide

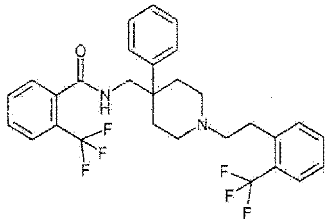

The compound of the subtitle (hereinafter, written as a compound 35 of the present invention) was obtained by performing the same reaction as that in Production Example 1 using 2-trifluoromethyl benzoic acid instead of 2,6-dichloroisonicotinic acid.
MS+:535.

(Production Example 36) Production of 2,4,6-trifluoro-N-((4-phenyl-1-(2-(trifluoromethyl)phenethyl)piperidin-4-yl)methyl)benzamide

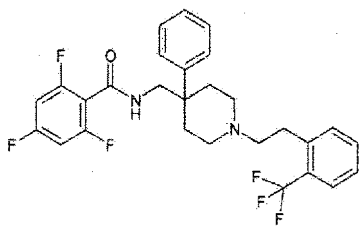

The compound of the subtitle (hereinafter, written as a compound 36 of the present invention) was obtained by performing the same reaction as that in Production Example 1 using 2,4,6-trifluorobenzoic acid instead of 2,6-dichloroisonicotinic acid.
$^1$H-NMR ($CDCl_3$) δ: 7.62-7.58 (1H, m), 7.47-7.42 (1H, m), 7.39-7.32 (5H, m), 7.30-7.23 (2H, m), 6.70-6.63 (2H, m), 5.48-5.38 (1H, m), 3.74-3.63 (2H, m), 3.01-2.90 (2H, m), 2.81-2.69 (2H, m), 2.62-2.53 (2H, m), 2.53-2.42 (2H, m), 2.32-2.22 (2H, m), 2.07-1.96 (2H, m).

(Production Example 37) Production of 2,3,5,6-tetrafluoro-N-((4-phenyl-1-(2-(trifluoromethyl)phenethyl)piperidin-4-yl)methyl)benzamide

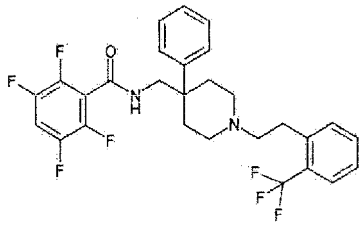

The compound of the subtitle (hereinafter, written as a compound 37 of the present invention) was obtained by performing the same reaction as that in Production Example 1 using 2,3,5,6-tetrafluorobenzoic acid instead of 2,6-dichloroisonicotinic acid.
$^1$H-NMR ($CDCl_3$) δ: 7.62-7.58 (1H, m), 7.48-7.31 (6H, m), 7.31-7.23 (2H, m), 7.13-7.04 (1H, m), 5.50-5.41 (1H, m), 3.74-3.68 (2H, m), 3.00-2.93 (2H, m), 2.80-2.71 (2H, m), 2.62-2.55 (2H, m), 2.53-2.44 (2H, m), 2.30-2.20 (2H, m), 2.08-2.00 (2H, m).

(Production Example 38) Production of 2,6-dichloro-N-((4-(pyridin-2-yl)-1-(2-(trifluoromethyl)phenethyl)piperidin-4-yl)methyl)isonicotinamide

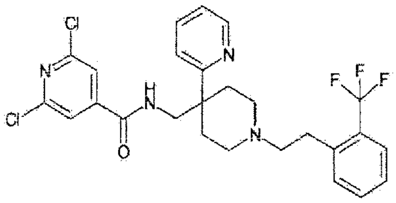

The compound of the subtitle (hereinafter, written as a compound 38 of the present invention) was obtained by performing the same reaction as that in Production Example 1 using 4-cyano-4-(pyridin-2-yl)piperidine hydrochloride instead of 4-cyano-4-phenylpiperidine hydrochloride.
$^1$H-NMR ($CDCl_3$) δ: 8.67-8.63 (1H, m), 8.18-8.12 (1H, m), 7.80-7.74 (1H, m), 7.64-7.59 (1H, m), 7.59-7.56 (2H, m), 7.49-7.43 (2H, m), 7.36-7.27 (3H, m), 3.73-3.68 (2H, m), 3.02-2.95 (2H, m), 2.76-2.67 (2H, m), 2.65-2.57 (2H, m), 2.56-2.47 (2H, m), 2.38-2.29 (2H, m), 2.01-1.90 (2H, m).

(Production Example 39) Production of 2,6-dichloro-N-((4-(pyridin-3-yl)-1-(2-(trifluoromethyl)phenethyl)piperidin-4-yl)methyl)isonicotinamide

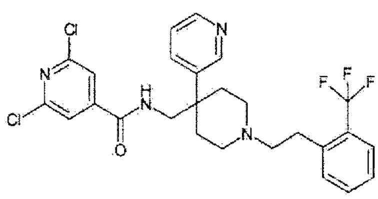

The compound of the subtitle (hereinafter, written as a compound 39 of the present invention) was obtained by performing the same reaction as that in Production Example 1 using 4-cyano-4-(pyridin-3-yl)piperidine hydrochloride instead of 4-cyano-4-phenylpiperidine hydrochloride.
$^1$H-NMR ($CDCl_3$) δ: 8.64-8.60 (1H, m), 8.53-8.49 (1H, m), 7.72-7.67 (1H, m), 7.65-7.58 (1H, m), 7.51-7.39 (3H, m),

7.39-7.28 (3H, m), 6.15-6.04 (1H, m), 3.73-3.66 (2H, m), 2.99-2.91 (2H, m), 2.84-2.75 (2H, m), 2.63-2.53 (2H, m), 2.47-2.40 (2H, m), 2.29-2.20 (2H, m), 2.10-2.01 (2H, m).

(Production Example 40) Production of 2-chloro-N-((4-(3-fluoropyridin-2-yl)-1-(2-(trifluoromethyl)phenethyl)piperidin-4-yl)methyl)isonicotinamide

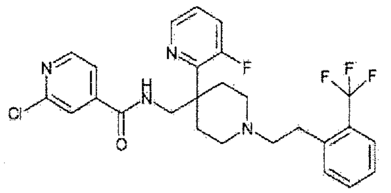

The compound of the subtitle (hereinafter, written as a compound 40 of the present invention) was obtained by performing the same reaction as that in Production Example 1 using 4-cyano-4-(3-fluoropyridin-2-yl)piperidine hydrochloride instead of 4-cyano-4-phenylpiperidine hydrochloride and using 2-chloroisonicotinic acid instead of 2,6-dichloroisonicotinic acid.
MS+:521.

(Production Example 41) Production of 2-chloro-N-((4-(pyridin-2-yl)-1-(2-(trifluoromethyl)phenethyl)piperidin-4-yl)methyl)benzamide

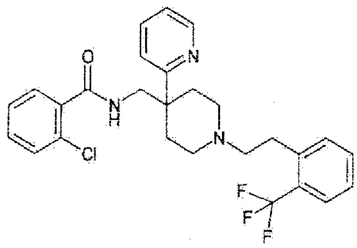

The compound of the subtitle (hereinafter, written as a compound 41 of the present invention) was obtained by performing the same reaction as that in Production Example 1 using 4-cyano-4-(pyridin-2-yl)piperidine hydrochloride instead of 4-cyano-4-phenylpiperidine hydrochloride and using 2-chlorobenzoic acid instead of 2,6-dichloroisonicotinic acid.
$^1$H-NMR ($CDCl_3$) δ: 8.57-8.54 (1H, m), 7.73-7.67 (1H, m), 7.63-7.59 (2H, m), 7.49-7.42 (2H, m), 7.37-7.27 (5H, m), 7.18-7.14 (1H, m), 7.07-7.01 (1H, m), 3.87-3.82 (2H, m), 3.03-2.96 (2H, m), 2.82-2.73 (2H, m), 2.67-2.52 (4H, m), 2.39-2.30 (2H, m), 2.05-1.96 (2H, m).

(Production Example 42) Production of 2-methoxy-N-((4-(pyridin-2-yl)-1-(2-(trifluoromethyl)phenethyl)piperidin-4-yl)methyl)benzamide

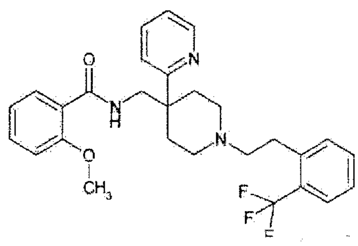

The compound of the subtitle (hereinafter, written as a compound 42 of the present invention) was obtained by performing the same reaction as that in Production Example 1 using 4-cyano-4-(pyridin-2-yl)piperidine hydrochloride instead of 4-cyano-4-phenylpiperidine hydrochloride and using 2-methoxybenzoic acid instead of 2,6-dichloroisonicotinic acid.
$^1$H-NMR ($CDCl_3$) δ: 8.68-8.64 (1H, m), 8.19-8.15 (1H, m), 8.14-8.09 (1H, m), 7.73-7.68 (1H, m), 7.62-7.59 (1H, m), 7.49-7.26 (5H, m), 7.21-7.17 (1H, m), 7.06-7.01 (1H, m), 6.92-6.86 (1H, m), 3.89-3.84 (2H, m), 3.76 (3H, s), 3.02-2.94 (2H, m), 2.81-2.73 (2H, m), 2.63-2.57 (2H, m), 2.56-2.46 (2H, m), 2.39-2.30 (2H, m), 2.06-1.97 (2H, m).

(Production Example 43) Production of 2-chloro-N-((4-phenyl)-1-(2,4-dichloro-benzyl)piperidin-4-yl)methyl)isonicotinamide

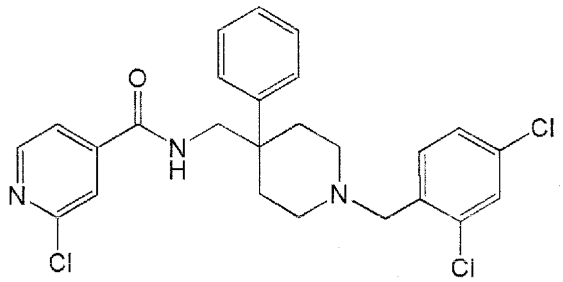

The compound of the subtitle (hereinafter, written as a compound 43 of the present invention) was obtained by performing the same reaction as that in Production Example 1 using 2,4-dichloro-benzyl methanesulfonate instead of 2-(trifluoromethyl)phenethyl methanesulfonate and using 2-chloroiscnicotinic acid instead of 2,6-dichloroisonicotinic acid.
MS+ : 488

(Production Example 44) Production of 2,6-dichloro-N-((4-phenyl)-1-(3-phenylpropyl)piperidin-4-yl)methyl)isonicotinamide

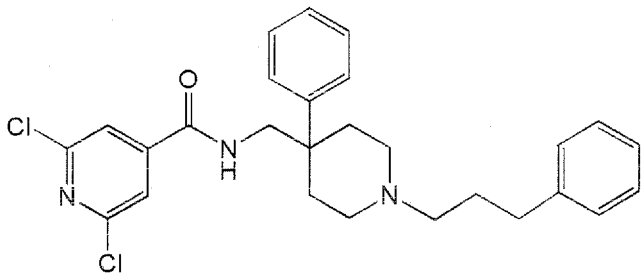

The compound of the subtitle (hereinafter, written as a compound 44 of the present invention) was obtained by performing the same reaction as that in Production Example 1 using 3-phenylpropyl methanesulfonate instead of 2-(trifluoromethyl)phenethyl methanesulfonate.
MS+ : 482

(Production Example 45) Production of 2,6-dichloro-N-((4-phenyl)-1-(4-phenyl-butyl)piperidin-4-yl)methyl)isonicotinamide

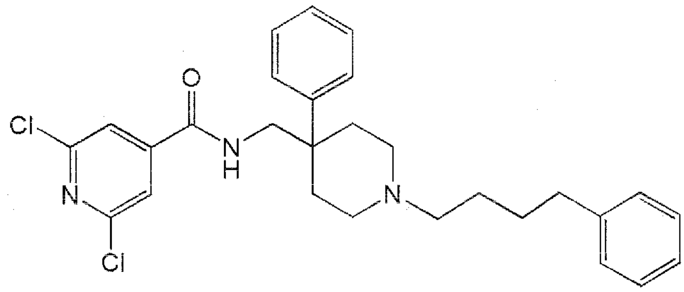

The compound of the subtitle (hereinafter, written as a compound 45 of the present invention) was obtained by performing the same reaction as that in Production Example 1 using 4-phenylbutyl methanesulfonate instead of 2-(trifluoromethyl)phenethyl methanesulfonate.
MS+ : 496

(Production Example 46) Production of 2-chloro-N-((4-phenyl)-1-(trans-3-(4-chlorophenyl)allyl)piperidin-4-yl)methyl)isonicotinamide

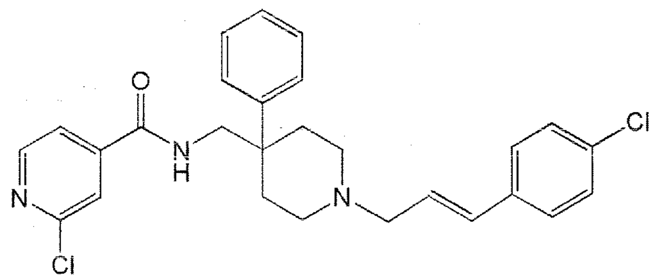

The compound of the subtitle (hereinafter, written as a compound 46 of the present invention) was obtained by performing the same reaction as that in Production Example 1 using 4-chloro-cinnamyl methanesulfonate instead of 2-(trifluoromethyl)phenethyl methanesulfonate and using 2-chloroisonicotinic acid instead of 2,6-dichloroiscnicotinic acid.
$^1$H-NMR (DMSO-$D_6$) δ: 8.55-8.51 (2H, m), 7.69 (1H, s), 7.61 (1H, d), 7.45-7.31 (7H, m), 7.20 (1H, t), 6.47 (1H, d), 6.31-6.25 (1H, m), 3.39-3.37 (2H, m), 2.98-2.95 (2H, m), 2.67-2.63 (2H, m), 2.19-2.14 (2H, m), 2.11-2.03 (2H, m), 1.92-1.86 (2H, m).
MS+ : 480

(Production Example 47) Production of 2-methyl-N-((4-phenyl-1-(2-(trifluoromethyl)phenethyl)piperidin-4-yl)methyl)benzamide

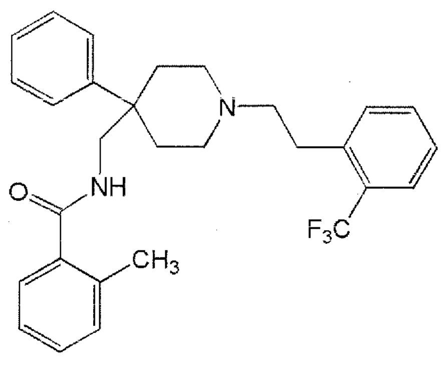

The compound of the subtitle (hereinafter, written as a compound 47 of the present invention) was obtained

by performing the same reaction as that in Production Example 1 using 2-chlorobenzoic acid instead of 2,6-dichloroisonicotinic acid.
$^1$H-NMR (DMSO-$D_6$) δ: 7.93 (1H, s), 7.65 (1H, d), 7.58 (1H, t), 7.48 (1H, d), 7.40 (3H, d), 7.35 (2H, dd), 7.27 (1H, t), 7.21-7.11 (4H, m), 3.40-3.31 (4H, m), 2.85 (2H, t), 2.71-2.67 (2H, m), 2.45-2.40 (2H, m), 2.21-2.16 (2H, m), 1.95-1.87 (2H, m).
MS+ : 481

(Production Example 48) Production of 2-trifluoromethoxy-N-((4-phenyl-1-(2-(trifluoromethyl)phenethyl)piperidin-4-yl)methyl)benzamide

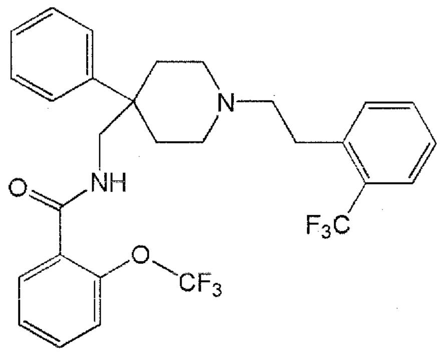

The compound of the subtitle (hereinafter, written as a compound 48 of the present invention) was obtained by performing the same reaction as that in Production Example 1 using 2-trifluoromethoxybenzoic acid instead of 2,6-dichloroisonicotinic acid.
$^1$H-NMR (DMSO-$D_6$) δ: 8.13 (1H, s), 7.65 (1H, d), 7.59-7.47 (3H, m), 7.43-7.38 (4H, m), 7.38-7.32 (4H, m), 7.22-7.17 (1H, m), 2.85 (2H, t), 2.71-2.67 (2H, m), 2.53-2.47 (2H, m), 2.43 (2H, t), 2.21-2.10 (4H, m), 1.96-1.89 (2H, m).
MS+ : 551

(Production Example 49) Production of 3-chloro-N-((4-phenyl-1-(2-(trifluoromethyl)phenethyl)piperidin-4-yl)methyl)pyrazine-6-carboxylic acid amide

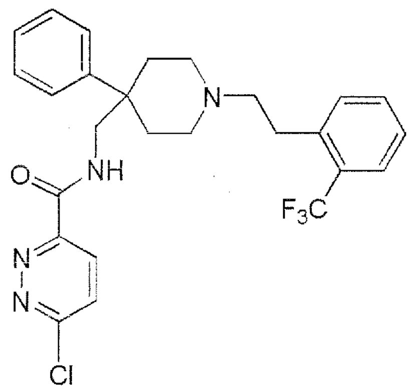

The compound of the subtitle (hereinafter, written as a compound 49 of the present invention) was obtained by performing the same reaction as that in Production Example 1 using 3-chloro-pyrazine-6-carboxylic acid instead of 2,6-dichloroisonicotinic acid.
$^1$H-NMR ($CDCl_3$) δ: 8.22 (1H, d), 7.74-7.25 (9H, m), 7.65 (1H, d), 3.65 (2H, d), 2.97-2.91 (2H, m), 2.80-2.72 (2H, m), 2.58-2.51 (2H, m), 2.47-2.35 (2H, m), 2.31-2.21 (2H, m), 2.08-1.97 (2H, m).

(Production Example 50) Production of 2,4-dichloro-N-((4-phenyl-1-(2-(trifluoromethyl)phenethyl)piperidin-4-yl)methyl)pyrimidine-6-carboxylic acid amide

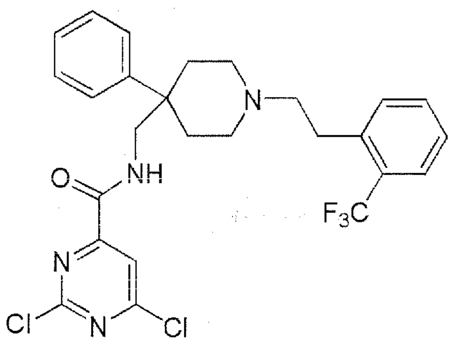

The compound of the subtitle (hereinafter, written as a compound 50 of the present invention) was obtained by performing the same reaction as that in Production Example 1 using 2,4-dichloro-pyrimidine-6-carboxylic acid instead of 2,6-dichloroisonicotinic acid.
$^{1}$H-NMR ($CDCl_3$) δ: 8.01 (1H, s), 7.59 (1H, d), 7.51-7.20 (9H, m), 3.60 (2H, d), 2.94 (2H, t), 2.75 (2H, brs), 2.58-2.56 (2H, m), 2.40 (2H, brs), 2.28-2.21 (2H, m), 2.01-1.99 (2H, m) .

(Production Example 51) Production of N-((4-phenyl-1-(2-(trifluoromethyl)phenethyl)piperidin-4-yl)methyl)pyrazine-2-carboxylic acid amide

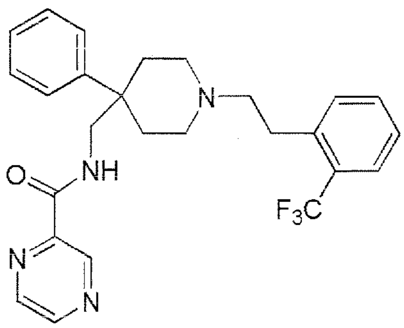

The compound of the subtitle (hereinafter, written as a compound 51 of the present invention) was obtained by performing the same reaction as that in Production Example 1 using pyrazine-2-carboxylic acid instead of 2,6-dichloroisonicotinic acid.
$^{1}$H-NMR ($CDCl_3$) δ: 9.36 (1H, d), 8.71 (1H, d), 8.43 (1H, m), 7.51-7.32(10H, m), 3.67 (2H, d), 2.95 (2H, t), 2.76 (2H, brs), 2.56 (2H, m), 2.44-2.42 (2H, m), 2.30-2.20 (2H, m), 2.06-2.00 (2H, m).

(Production Example 52) Production of 1-methyl-N-((4-phenyl-1-(2-(trifluoromethyl)phenethyl)piperidin-4-yl)methyl)pyrazole-4-carboxylic acid amide

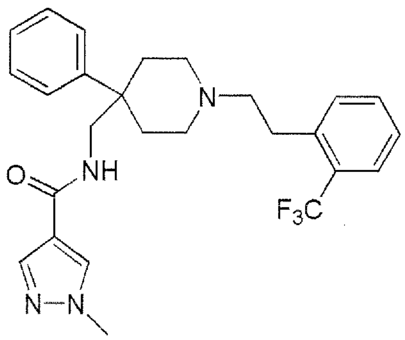

The compound of the subtitle (hereinafter, written as a compound 52 of the present invention) was obtained by performing the same reaction as that in Production Example 1 using 1-methyl-pyrazole-4-carboxylic acid instead of 2,6-dichloroisonicotinic acid.
$^1$H-NMR ($CDCl_3$) δ: 7.69-7.26 (11H, m), 5.23-5.21 (1H, m), 3.89 (3H, s), 3.59 (2H, d), 2.95 (2H, t), 2.80-2.70 (2H, m), 2.58-2.53 (2H, m), 2.50-2.40 (2H, m), 2.22-2.14 (2H, m), 2.06-1.94 (2H, m).

(Production Example 53) Production of 1-methyl-N-((4-phenyl-1-(2-(trifluoromethyl)phenethyl)piperidin-4-yl)methyl)imidazole-4-carboxylic acid amide

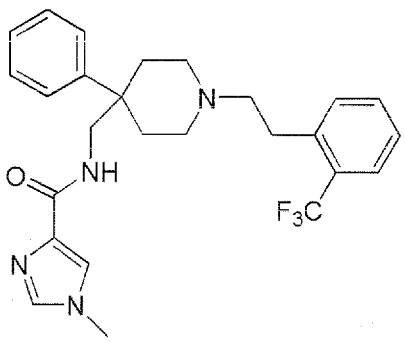

The compound of the subtitle (hereinafter, written as a compound 53 of the present invention) was obtained by performing the same reaction as that in Production Example 1 using 1-methylimidazole-4-carboxylic acid instead of 2,6-dichloroisonicotinic acid.
$^1$H-NMR ($CDCl_3$) δ: 7.59 (1H, d), 7.45-7.21 (10H, m), 6.80 (1H, brs), 3.87 (3H, s), 3.64 (2H, d), 2.96-2.92 (2H, m), 2.78-2.73 (2H, m), 2.58-2.55 (2H, m), 2.48-2.35 (2H, m), 2.28-2.16 (2H, m), 2.08-1.97 (2H, m).

(Production Example 54) Production of 1-methyl-N-((4-phenyl-1-(2-(trifluoromethyl)phenethyl)piperidin-4-yl)methyl)pyrrole-2-carboxylic acid amid

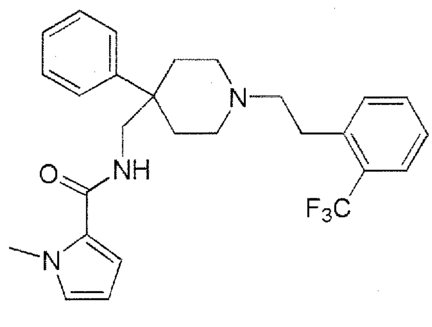

The compound of the subtitle (hereinafter, written as a compound 54 of the present invention) was obtained

by performing the same reaction as that in Production Example 1 using 1-methylpyrrole-2-carboxylic acid instead of 2,6-dichloroisonicotinic acid.
$^1$H-NMR ($CDCl_3$) δ: 7.59 (1H, d), 7.45-7.26 (8H, m), 6.67 (1H, t), 6.17(1H, m), 6.00(1H, m), 5.40(1H, t), 3.80 (3H, s), 3.57 (2H, d), 3.04-2.93 (2H, m), 2.78-2.68 (2H, m), 2.60-2.54 (2H, m), 2.48 (2H, brs), 2.24-2.13 (2H, m), 2.06-1.96 (2H, m).

Next, formulation examples of the compound of the present invention will be shown. Fart(s) represents part(s) by weight.

(Formulation Example 1)

10 parts of any one of the compounds 1 to 54 of the present invention is dissolved in a mixture of 35 parts of xylene and 35 parts of N,N-dimethylformamide, 14 parts of polyoxyethylene styryl phenyl ether and 6 parts of calcium dodecylbenzene sulfonate are added thereto, and the mixture is mixed to obtain each emulsion.

(Formulation Example 2)

4 parts of sodium lauryl sulfate, 2 parts of calcium lignin sulfonate, 20 parts of synthetic hydrated silicon oxide fine powder, and 54 parts of diatomaceous earth are mixed, 20 parts of any one of the compounds 1 to 54 of the present invention is further added thereto, and the mixture is mixed to obtain each a water-dispersible agent.

(Formulation Example 3)

1 part of synthetic hydrated silicon oxide fine powder, 2 parts of calcium lignin sulfonate, 30 parts of bentonite, and 65 parts of kaolin clay are added to 2 parts of any one of the compounds 1 to 54 of the present invention, and the mixture is mixed. Then, a suitable amount of water is added to the mixture, further stirred, granulated using a granulator, and air dried to obtain each granule.

(Formulation Example 4)

1 part of any one of the compounds 1 to 54 of the present invention is dissolved in a suitable amount of acetone, 5 parts of synthetic hydrated silicon oxide fine powder, 0.3 parts of PAP, and 93.7 parts of Fubasami clay are added thereto. The mixture is sufficiently stirred and mixed and acetone is removed by evaporation to obtain each powder.

(Formulation Example 5)

35 parts of a mixture (weight ratio of 1:1) of polyoxyethylene alkyl ether sulfate ammonium salt and white carbon, 10 parts of any one of the compounds 1 to 54 of the present invention, and 55 parts of water are mixed and pulverized through a wet pulverization method to obtain each formulation.

(Formulation Example 6)

0.1 parts of any one of the compounds 1 to 54 of the present invention is dissolved in 5 parts of xylene and 5 parts of trichloroethane, and the mixture is mixed with 89.9 parts of deodorized kerosine to obtain each oil solution.

(Formulation Example 7)

10 mg of any one of the compounds 1 to 54 of the present invention is dissolved in 0.5 ml of acetone, and the solution is treated to 5 g of animal solid feed powder (CE-2: solid feed powder for growing and breeding, manufactured by CLEA Japan, Inc.) and uniformly mixed. Next, acetone is dried by evaporation to obtain each poison bait.

(Formulation Example 8)

0.1 parts of any one of the compounds 1 to 54 of the present invention and 49.9 parts of Neo-chioZol (Chuo Kasei Co., Ltd.) are put into an aerosol can which is then installed with an aerosol valve. Thereafter, the can is filled with 25 parts of dimethyl ether and 25 parts of LPG, vibration is applied to the can, and an actuator is installed to obtain an oil aerosol.

(Formulation Example 9)

An aerosol container is filled with a mixture in which 0.6 parts of any one of the compounds 1 to 54 of the present invention, 0.01 parts of BHT (2,6-di-tert-butyl-4-methylphenol), 5 parts of xylene, 3.39 parts of deodorized kerosene, and 1 part of emulsifier {ATMOS 300 (registered trade name of ATMOS Chemical Ltd.)} are mixed together and dissolved, and is installed with a valve. Then, the container is filled with 40 parts of propellant (LPG) under pressure through the valve to obtain an aqueous aerosol.
Next, the efficacy of the compound of the present invention in controlling pests will be shown with reference to test examples.

(Test Example 1) Controlling Effect on Cotton Aphid

Spray solutions for a test were prepared by diluting each formulation of the compounds 2, 3, 11, 12, 16, 20, 21, 24 to 29, 36, and 38 to 42 of the present invention, which were obtained from Formulation Example 5, with water so that the concentration of the active ingredients becomes 500 ppm.
Cucumber seedlings (first-leaf development stage) planted in plastic cups were inoculated with about 30 cotton aphids and were allowed to stand for 1 day. Each 20 ml of the diluted solutions was sprayed on the seedlings.
6 days after the spraying, the number of surviving parasitic cotton aphids on the leaves of the cucumber was checked and the controlling value was obtained using the following formula.

Controlling value (%) = {1 - (Cb x Tai) / (Cai x Tb)} x 100

The characters in the formula represent the following meanings.

Cb: the number of insects in a non-treatment section before treatment
Cai: the number of insects in a non-treatment section on observation
Tb: the number of insects in a treatment section before treatment
Tai: the number of insects in a treatment section on observation

Herein, the non-treatment section refers to a section in which a liquid medicine for a test which has been obtained by diluting each of formulations which do not contain the compounds of the present invention in Formulation Example 5 with the same amount of water as that in the treatment section has been sprayed.
As a result, each treatment section of the testing spray solution of each of the compounds of the present invention, which were provided for the test, showed a controlling effect higher than or equal to 90%.

(Test Example 2) Controlling Effect on Summer Fruit Tortrix Moth

Liquid medicines for a test were prepared by diluting each formulation of the compounds 1, 3, 6, 10, 12, 16, 17, 20, 21, 23, 26 to 29, 33, 38, and 42 of the present invention, which were obtained from Formulation Example 5, with water so that the concentration of the active ingredients becomes 500 ppm.
Silk Mates 2S (arfiticial feed: Nosan Corporation), which were sliced in a thickness of 2 mm, were placed on the bottom of polyethylene cups and 1 mL of each of the above-described liquid medicines for a test was irrigated thereto.
After air-drying, 30 first-instar larvae of summer fruit tortrix moths (Adoxophyes orana) were released in each cup, which was then covered with a lid. The cups were stored at 25°C, the number of surviving insects was counted after 7 days, and the mortality rate was obtained using the following formula.

Mortality (%) = (the number of dead insects / the number of insects provided for a test) x 100

As a result, each treatment section of the testing liquid medicine of each of the compounds of the present invention, which were provided for the test, showed mortality higher than or equal to 90%.

(Test Example 3) Controlling Effect on Oriental Leafworm Moth

Liquid medicines for a test were prepared by diluting each formulation of the compounds 1, 13, 17, and 33 of the present invention, which were obtained from Formulation Example 5, with water so that the concentration of the active ingrediants becomes 500 ppm.

Insecta LFs (arfiticial feed: Nosan Corporation), which were cut into a semicircular shape with a thickness of 6 mm, were placed on the bottom of polyethylene cups and 2 mL of each of the above-described liquid medicines for a test was irrigated thereto.

After air-drying, 5 fourth-instar larvae of oriental leafworm moth (Spodoptela litura) were released in each cup, which was then covered with a lid. The cups were stored at 25°C, the number of surviving insects was counted after 6 days, and the mortality rate was obtained using the following formula.

Mortality (%) = (the number of dead insects / the number of insects provided for a test) x 100

As a result, each treatment section of the testing liquid medicine of each of the compounds of the present invention, which were provided for the test, showed mortality higher than or equal to 80%.

(Test Example 4) Controlling Effect on Two-spotted Spider Mite

Liquid medicines for a test were prepared by diluting each formulation of the compounds 1 and 38 of the present invention, which were obtained from Formulation Example 5, with water so that the concentration of the active ingredients becomes 500 ppm.

Bush bean seedlings in a primary leaf development stage which were planted in polyethylene cups were inoculated with about 60 female imagoes of two-spotted spider mites. Then, 30 ml of each of the above-described diluted solutions was sprayed on the seedlings one day after the inoculation.

8 days after the spraying, the number of surviving parasitic mites on the leaves of the bush bean was checked and the controlling rate was obtained using the following formula.

Controlling rate (%) = 100 x {1 - (the number of surviving mites in a treatment section) / (the number of surviving mites in a non-treatment section))}

As a result, each treatment section of the testing spray solution of each of the compounds of the present invention, which were provided for the test, showed a controlling rate higher than or equal to 90%.

(Test Example 5) Controlling Effect on Brown Planthopper

Liquid medicines for a test were prepared by diluting each formulation of the compounds 1, 26 and 27 of the present invention, which were obtained from Formulation Example 5, with water so that the concentration of the active ingredients becomes 500 ppm.

10 ml of the above-described liquid solution for a test was sprayed on rice seedlings in a second-leaf development stage which were planted in polyethelene cups. After air-drying, 20 third- to fourth-instar larvae of the brown planthoppers were released in the cup and stored at a room temperature of 25°C. The number of surviving parasitic brown planthoppers on the rice was checked after 6 days and the controlling value was obtained using the following formula.

Controlling value (%) = {1 - (Cb x Tai) / (Cai x Tb)} x 100

The characters in the formula represent the following meanings.

Cb: the number of insects in a non-treatment section before treatment
Cai: the number of insects in a non-treatment section on observation
Tb: the number of insects in a treatment section before treatment
Tai: the number of insects in a treatment section on observation

As a result, each treatment section of the testing spray solution of each of the compounds of the present invention, which were provided for the test, showed a controlling value higher than or equal to 90%.

(Test Example 6) Controlling Effect on Diamondback Moth

Spray solutions for a test were prepared by diluting each formulation of the compounds 1, 11 to 14, 16, 17, 36, and 38 of the present invention, which were obtained from Formulation Example 5, with water so that the concentration of the active ingredients becomes 500 ppm.

Meanwhile, each of the above-described liquid solutions for the test was sprayed on cabbages in a third-leaf stage which were planted in polyethylene cups at a ratio of 20 mL/cup. After the liquid solutions were dried, the stems and leaves were cut and accommodated in each 50 m L cup. 5 second-instar larvae of diamondback moths were released in each cup, which was then covered with a lid. The cups were stored at 25°C, the number of surviving insec ts was counted after 5 days, and the mortality rate was obtained using the following formula.

Mortality (%) = (the number of dead insects / the number of insects provided for a test) x 100

As a result, each treatment section of the testing spray solution which contains each of the compounds of the present invention, which were provided for the test, showed a mortality rate higher than or equal to 80%.

(Test Example 7) Controlling Effect on Common Housefly

Liquid medicines for a test were prepared by diluting a formulation of the compound 28 of the present invention, which was obtained from Formulation Example 5, with water so that the concentration of the active ingredients becomes 500 ppm.

A filter paper having a diameter of 5.5 cm was spread on the bottom of a polyethylene cup having the same size as that of the filter paper, 0.7 ml ml of any one of the above-described liquid medicines for a test was added dropwise onto the filter paper, and 30 mg of sucrose was uniformly put therein as bait. 10 female imagoes of common houseflies were released in the polyethylene cup, which was then covered with a lid. The number oflive and dead common houseflies were checked after 24 hours and the mortality rate was obtained using the following formula.

Mortality (%) = (the number of dead insectse / the number of insects provided for a test) x 100

As a result, the treatment section of the testing liquid medicine of the compound of the present invention, which was provided for the test, showed a mortality rate higher than or equal to 70%.

Industrial Applicability

The compound of the present invention has an excellent controlling efficacy against pests and is effective as active ingredients of a pest control agent.

## Claims

1. A piperidine compound represented by Formula (I):

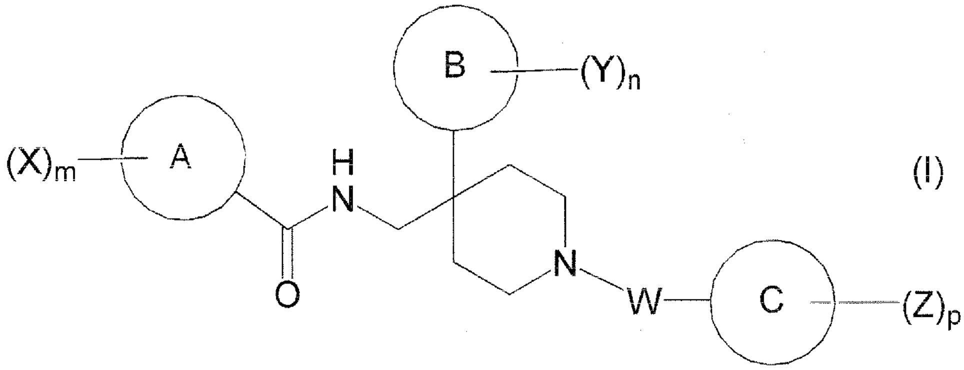

(I)

wherein ring A, ring B, and ring C are the same or differen t and each represent a benzene ring or a nitrogen-containing heteroaromatic ring; W represents a C1-C4 alkylene or alkenylene group; X, Y, and Z are the same or d ifferent and each represent a halogen atom, a C1-C6 alkyl group, a halo C1-C6 alkyl group, a C1-C6 alkoxy group, or a halo C1-C6 alkoxy group; m, n, and p are the same or differe nt and each represent an integer of 0 to 5; when m is greater than or equal to 2, the Xs are the same or different; when n is greater than or equal to 2, the Ys are the same or different; and when p is greater than or equal to 2, the Zs are the same or different.

2. The piperidine compound according to claim 1, wherein W is an ethylene group.

3. The piperidine compound according to claim 1 or 2, wherein the nitrogen-containing heteroaromatic ring is a pyrrole ring, an imidazole ring, a pyrazole ring, a pyridine ring, a pyrazine ring, a pyrimidine ring, or a pyridazine ring.

4. The piperidine compound according to claim 1 or 2, wherein the group represented by the following Formula:

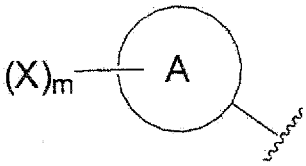

wherein ring A, X, and m are as defined above
is a group represented by any one of the following formulas

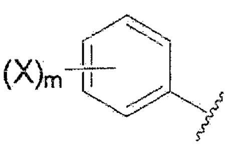 , 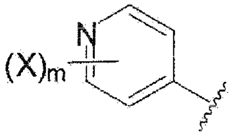 , 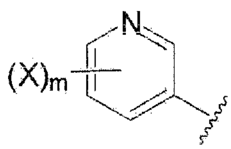 or 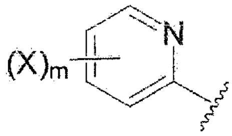 ,

the group represented by the following formula:

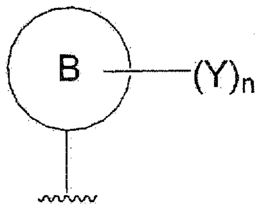

is a group represented by any one of the following formulas

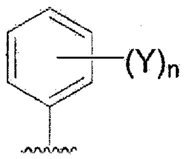 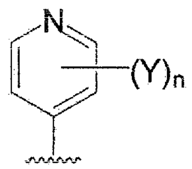 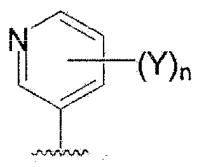 or 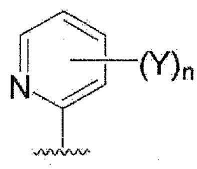,

and the group represented by the following formula:

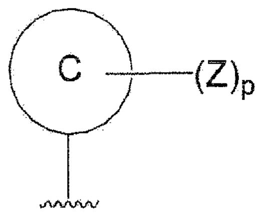

is a group represented by any one of the following Formulas

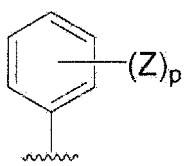 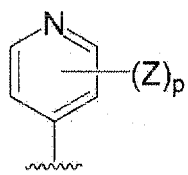 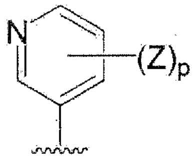 or 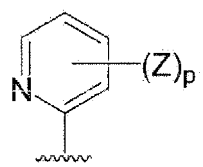.

**5.** The piperidine compound according to claim 1 or 2, wherein the group represented by the following Formula:

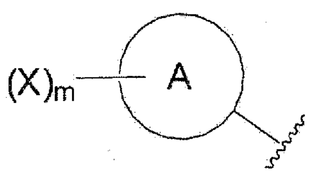

is a phenyl group, a 2-halophenyl group, a 3-halophenyl group, a 4-halophenyl group, a 3,5-dihalophenyl group, a 2,4,6-trihalophenyl group, a 2,3,5,6-tetrahalophenyl group, a 1-methoxyphenyl group, a 2-methoxyphenyl group, a 3-methoxyphenyl group, a 2,6-dimethoxyphenyl group, a 1-trifluoromethoxyphenyl group, a 2-trifluoromethoxyphenyl group, and a 3-trifluoromethoxyphenyl group; a 2-pyridyl group, a 3-pyridyl group, a 4-pyridyl group, a 2-halopyridin-4-yl group, a 2,6-dihalopyridin-4-yl group, a 3-halopyridin-2-yl group, or a 2-halopyridin-3-yl group, the group represented by the following Formula:

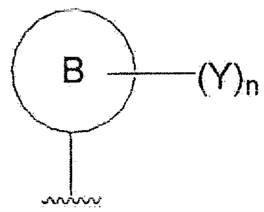

is a phenyl group, a 2-halophenyl group, a 3-halophenyl group, a 4-halophenyl group, a 3,5-dihalophenyl group, a 2,4,6-trihalophenyl group, a 2,3,5,6-tetrahalophenyl group, a 1-methoxyphenyl group, a 2-methoxyphenyl group, a 3-methoxyphenyl group, a 2,6-dimethoxyphenyl group, a 1-trifluoromethoxyphenyl group, a 2-trifluoromethoxyphenyl group, and a 3-trifluoromethoxyphenyl group; a 2-pyridyl group, a 3-pyridyl group, a 4-pyridyl group, a 2-halopyridin-4-yl group, a 2,6-dihalopyridin-4-yl group, a 3-halopyridin-2-yl group, or a 2-halopyridin-3-yl group, and the group represented by the following Formula:

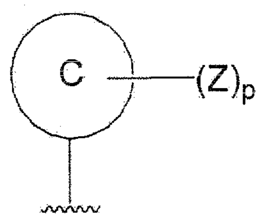

is a phenyl group, a 2-halophenyl group, a 3-halophenyl group, a 4-halophenyl group, a 3,5-dihalophenyl group, a 2,4,6-trihalophenyl group, a 2,3,5,6-tetrahalophenyl group, a 1-methoxyphenyl group, a 2-methoxyphenyl group, a 3-methoxyphenyl group, a 2,6-dimethoxyphenyl group, a 1-trifluoromethoxyphenyl group, a 2-trifluoromethoxyphenyl group, and a 3-trifluoromethoxyphenyl group; a 2-pyridyl group, a 3-pyridyl group, a 4-pyridyl group, a 2-halopyridin-4-yl group, a 2,6-dihalopyridin-4-yl group, a 3-halopyridin-2-yl group, or a 2-halopyridin-3-yl group.

**6.** A pest control agent comprising the piperidine compound according to claim 1 or 2 and an inert carrier.

**7.** A method for controlling pests comprising applying an effective amount of the piperidine compound according to claim 1 or 2 to pests or a habitat of the pests.

## INTERNATIONAL SEARCH REPORT

International application No.
PCT/JP2013/061559

**A. CLASSIFICATION OF SUBJECT MATTER**

*C07D211/26*(2006.01)i, *A01N43/40*(2006.01)i, *A01P7/02*(2006.01)i, *A01P7/04*(2006.01)i, *C07D401/04*(2006.01)i, *C07D401/12*(2006.01)i, *C07D401/14*(2006.01)i

According to International Patent Classification (IPC) or to both national classification and IPC

**B. FIELDS SEARCHED**

Minimum documentation searched (classification system followed by classification symbols)
C07D211/26, A01N43/40, A01P7/02, A01P7/04, C07D401/04, C07D401/12, C07D401/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus/REGISTRY(STN), JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2003/088908 A2 (Bristol-Myers Squibb Co.), 30 October 2003 (30.10.2003), examples 657, 662, 684, 685<br>& JP 2005-529114 A & JP 2010-215666 A<br>& JP 4729259 B & US 2004/0110793 A1<br>& US 2005/0171156 A1 & US 2006/0014792 A1<br>& US 2009/0312307 A1 & EP 1501467 A<br>& EP 2228065 A2 & EP 2371366 A1<br>& EP 1841741 A & WO 2006/073967 A1 | 1,4,5<br>2,3,6,7 |
| X<br>A | WO 2000/025786 A1 (Merck & CO., INC.), 11 May 2000 (11.05.2000), example 1<br>& JP 2002-528503 A & JP 4764550 B<br>& US 6303637 B1 & EP 1126849 A | 1,4,5<br>2,3,6,7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>16 May, 2013 (16.05.13) | Date of mailing of the international search report<br>28 May, 2013 (28.05.13) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No. PCT/JP2013/061559

C (Continuation). DOCUMENTS CONSIDERED TO BE RELEVANT

| Category* | Citation of document, with indication, where appropriate, of the relevant passages | Relevant to claim No. |
|---|---|---|
| A | WO 2008/124757 A1 (Bristol-Myers Squibb Co.), 16 October 2008 (16.10.2008), example 6<br>& JP 2010-523693 A & US 2010/0120770 A1<br>& EP 2132203 A & CN 101679406 A | 1-7 |
| A | WO 2001/007050 A1 (Schering Corp.), 01 February 2001 (01.02.2001), table 1, 15th, 27th compounds<br>& JP 2003-505420 A & JP 2009-513655 A<br>& US 6262066 B1 & US 2001/0011092 A1<br>& US 2003/0073690 A1 & US 2004/0067950 A1<br>& US 2004/0152707 A1 & US 2006/0217414 A1<br>& EP 1200087 A & EP 1951720 A | 1-7 |
| A | US 3455940 A (Stecker International S.p.A.), 15 July 1969 (15.07.1969), table 1, no.15<br>& GB 1167734 A & DE 1593552 A<br>& FR 1501151 A | 1-7 |
| A | WO 2006/003494 A2 (Syngenta Participations AG), 12 January 2006 (12.01.2006), formula (I); particularly, examples 1, 4, 5, 12; claims 1 to 12<br>& JP 2008-504253 A & JP 5043653 B<br>& US 2009/0042938 A1 & US 2012/0270885 A1<br>& GB 414438 D & GB 414438 D0<br>& EP 1763302 A & KR 10-2007-0029214 A<br>& CN 1976584 A | 1-7 |
| A | WO 2005/036961 A2 (FMC Corp.), 28 April 2005 (28.04.2005), formula (I); claims 1 to 40<br>& JP 2007-508306 A & JP 2008-512488 A<br>& US 2009/0215821 A1 & US 2008/0039631 A1<br>& EP 1673083 A & EP 1791828 A<br>& KR 10-2006-0119996 A & CN 1867330 A | 1-7 |
| A | WO 2001/017965 A2 (Syngenta Participations AG), 15 March 2001 (15.03.2001), formula (I); claims 1 to 7<br>& AU 7514700 A | 1-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Arch. Pharm. (Weinheim),* 1986, vol. 319, 505-515